(19)

Europäisches
Patentamt
European
Patent Office
Office européen
des brevets

(11) **EP 4 431 523 A1**

(12) **EUROPEAN PATENT APPLICATION**
published in accordance with Art. 153(4) EPC

(43) Date of publication:
**18.09.2024 Bulletin 2024/38**

(21) Application number: **22891989.0**

(22) Date of filing: **08.11.2022**

(51) International Patent Classification (IPC):
*C07K 14/705* (2006.01)   *C12N 15/10* (2006.01)
*C12N 15/63* (2006.01)   *A61P 35/00* (2006.01)
*A61P 17/00* (2006.01)   *A61P 9/10* (2006.01)
*A61P 13/12* (2006.01)   *A61P 15/00* (2006.01)

(52) Cooperative Patent Classification (CPC):
**A61K 35/17; A61P 9/10; A61P 13/12; A61P 15/00;
A61P 17/00; A61P 35/00; C07K 14/705;
C07K 19/00; C12N 5/10; C12N 15/10; C12N 15/62;
C12N 15/63; C12N 15/85**

(86) International application number:
**PCT/CN2022/130701**

(87) International publication number:
**WO 2023/083195 (19.05.2023 Gazette 2023/20)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **09.11.2021   CN 202111322040**

(71) Applicant: **West China Hospital, Sichuan
University
Chengdu, Sichuan 610041 (CN)**

(72) Inventors:
• **ZHAO, Xudong**
  **Chengdu, Sichuan 610041 (CN)**
• **FAN, Jiawei**
  **Chengdu, Sichuan 610041 (CN)**

(74) Representative: **Santoro, Sofia et al
Società Italiana Brevetti S.p.A.
Via Giosuè Carducci, 8
20123 Milano (IT)**

Remarks:
The complete document including Reference
Table(s) and the Sequence Listing(s) can be
downloaded from the EPO website

(54) **PREPARATION FOR CHIMERIC ANTIGEN RECEPTOR IMMUNE CELL CONSTRUCTED ON BASIS OF GAS6 AND USE OF CHIMERIC ANTIGEN RECEPTOR IMMUNE CELL**

(57)   Provided in the present invention are the preparation for a chimeric antigen receptor immune cell constructed on the basis of a growth arrest-specific protein 6 and the use of the chimeric antigen receptor immune cell. Specifically, provided in the present invention is a chimeric antigen receptor (CAR) based on GAS6 transformation. The CAR contains an extracellular binding domain, which can specifically target a GAS6 receptor. The CAR immune cell of the present invention has a strong specificity and target affinity, thereby also having a strong target cell killing capability and a high safety.

EP 4 431 523 A1

## Description

### Technical field

The present invention belongs to the art of immune cell therapy, and specifically relates to the preparation and application of a chimeric antigen receptor immune cell constructed on basis of GAS6.

### Background

Tumor is the second major disease that threatens human health. In 2018, there were 18,100,000 new cancer patients and 9,500,000 tumor deaths worldwide. It is estimated that by 2040, there will be 29,500,000 new cases and 16,400,000 deaths of cancer per year. Although conventional tumor treatment methods such as radiotherapy, chemotherapy, and surgical resection are able to prolong the survival of cancer patients, they are still constrained by the decline in life quality of patients and high risk of recurrence.

Bioimmunotherapy has become the fourth cancer treatment method after surgery, radiotherapy and chemotherapy, and will become a must-have method for future cancer treatment. Chimeric Antigen Receptor-T cells (CAR-T) refer to T cells that are genetically modified to be able to recognize specific target antigens in a non MHC-restricted manner, with continuous activation and amplification. The structure of CAR comprises a tumor associated antigen binding region, an extracellular hinge region, a transmembrane region and an intracellular signaling region. At present, CART therapy has shown good therapeutic effect on hematological malignancies, but the application of CART therapy in solid tumors is limited due to tumor heterogeneity, lack of tumor specific antigens, and tumor immunosuppressive microenvironment.

Numerous studies have demonstrated that AXL, a member of the Receptor tyrosine kinase (RTK) TAM family, is involved in various cellular processes, including cell growth, proliferation, survival, apoptosis, and adhesion. AXL is highly expressed in various malignant tumors ranging from solid tumors to hematologic malignancies, including solid tumors such as lung cancer, pancreatic cancer, glioma, prostate cancer, breast cancer, liver cancer, colon cancer, ovarian cancer, gastric cancer, nasopharyngeal cancer, etc., as well as hematologic malignancies such as acute myeloid leukemia (AML), multiple myeloma (MM), chronic lymphocytic leukemia (CLL), acute lymphoblastic leukemia (ALL), and diffuse large B-cell lymphoma (DLBCL), which all highly express AXL that promotes tumor formation. Concurrently, researches have found that AXL expression is upregulated in macrophages in inflammatory diseases or associated with tumor microenvironments. Therefore, targeting AXL has the ability for inhibiting inflammation or anti-tumor. AXL is highly expressed in primary tumors and metastases compared with that in normal tissues, which is associated with poor prognosis and chemoresistance in multiple cancers. The novel role of AXL in biological processes has made it a tumor biomarker and a marker for tumorigenesis.

Pancreatic ductal adenocarcinoma (PDAC) is the most common type of pancreatic cancer and is expected to become one of the leading causes of cancer deaths in the United States by 2030. Despite progress in systemic chemotherapy for metastatic PDAC, the overall survival rate for this disease remains low. The main characteristics of pancreatic cancer are metastasis, treatment resistance, and immunosuppression. AXL, a member of the RTK TAM family, is associated with the plasticity, chemoresistance, immunosuppression, and metastasis of pancreatic cancer cells. Research has shown that the expression of receptor tyrosine kinase AXL is significantly increased in tumor tissues of approximately 70% of pancreatic cancer patients, while it is almost unexpressed in normal pancreatic duct epithelial cells. In cancer patients, overexpression of AXL protein is positively correlated with lymph node metastasis, tumor drug resistance, distant metastasis, disease progression, poor overall survival, and recurrence rates. Studies have shown that knocking down the expression of AXL increases the sensitivity of pancreatic cancer cells to gemcitabine.

The above studies indicate that AXL is an important target for cancer treatment; targeting AXL can effectively inhibit the growth, invasion, metastasis, and drug resistance of a tumor, thereby prolonging the survival rate of a patient.

Therefore, there is an urgent need to develop a chimeric antigen receptor immune cell targeting AXL and a therapeutic method using the same in this field.

### Summary of the invention

The purpose of the invention is to provide a chimeric antigen receptor immune cell targeting GAS6 receptor and the preparation and application method thereof.

In the first aspect of the present invention, it provides a chimeric antigen receptor (CAR) comprising an extracellular binding domain which comprises a structure based on a GAS6 protein comprising an amino acid sequence set forth in SEQ ID NO: 1 or a fragment thereof and is able to specifically bind to a GAS6 receptor.

In another preferred embodiment, the extracellular binding domain comprises an amino acid sequence derived from GAS6.

In another preferred embodiment, the extracellular binding domain comprises the GAS6 protein or a fragment thereof.

In another preferred embodiment, the extracellular binding domain comprises a extracellular domain of the GAS6 protein.

In another preferred embodiment, the extracellular binding domain comprises a laminin G-like (LG) domain of the GAS6 protein.

In another preferred embodiment, the LG domain comprises a LG1 doamin and a LG2 domain.

In another preferred embodiment, the LG domain includes wild-type and mutated LG domain.

In another preferred embodiment, the amino acid sequence of the LG domain is shown in positions X to 678 of the sequence of SEQ ID NO: 1, wherein X is a positive integer of any one of 250-285, preferably 260-279, and most preferably 261.

In another preferred embodiment, the amino acid sequence of the LG domain is shown in positions 279 to 678 of the sequence of SEQ ID NO: 1.

In another preferred embodiment, the GAS6 protein or the fragment thereof comprises a laminin G-like (LG) domain of the GAS6 protein, the amino acid sequence of which corresponds to positions 279-678 of the sequence shown in SEQ ID NO: 1.

In another preferred embodiment, the GAS6 protein or the fragment thereof comprises an epidermal growth factor (EGF)-like domain of the GAS6 protein, the amino acid sequence of which is set forth in positions 118-278 of the sequence shown in SEQ ID NO: 1.

In another preferred embodiment, the amino acid sequence of the EGF-like domain is shown in positions n to 278 of the sequence of SEQ ID NO: 1,

wherein n is a positive integer of any one of 118-275, preferably 250-270, and most preferably 261.

In another preferred embodiment, the GAS6 protein or the fragment thereof comprises an extracellular domain of the GAS6 protein, the amino acid sequence of which corresponds to positions 31-678 of the sequence shown in SEQ ID NO: 1.

In another preferred embodiment, the extracellular binding domain of the CAR further comprises a second extracellular domain targeting another target besides the first extracellular domain targeting a GAS6 receptor.

In another preferred embodiment, the another target is a tumor-specific target.

In another preferred embodiment, the GAS6 protein or the fragment thereof specifically binds to a GAS6 receptor including an AXL protein.

In another preferred embodiment, the GAS6 receptor is a receptor tyrosine kinase.

In another preferred embodiment, the receptor tyrosine kinase is selected from a group consisting of: AXL, Tyro3, MERTK, and a combination thereof.

In another preferred embodiment, the GAS6 receptor is selected from a group consisting of: AXL, Tyro3 and MERTK.

In another preferred embodiment, the receptor tyrosine kinase AXL is on the cell membrane (or transmembrane, or in the cytoplast).

In another preferred embodiment, the receptor tyrosine kinase is an AXL.

In another preferred embodiment, the receptor tyrosine kinase AXL is derived from human or a non-human mammal.

In another preferred embodiment, the non-human mammal includes: a rodent (such as a rat, a mouse), a primate (such as a monkey); and preferably is a primate.

In another preferred embodiment, the receptor tyrosine kinase AXL is of human or monkey origin.

In another preferred embodiment, the AXL is of human origin.

In another preferred embodiment, the GAS6 protein or the fragment thereof has an amino acid sequence as shown in SEQ ID NO: 1, or has an amino acid sequence as shown in positions 1 to 678 (preferably positions 31 to 678, more preferably positions 118-678, and more preferably positions 261-678) of SEQ ID NO: 1.

In another preferred embodiment, the amino acid sequence of the GAS6 protein or the fragment thereof is selected from a group consisting of:

(i) a sequence as shown in positions 31-678 of the sequence of SEQ ID NO: 1; and
(ii) on the basis of the sequence shown in positions 261-678 of the sequence of SEQ ID NO: 1, an amino acid sequence obtained by replacing, deleting, altering or inserting one or more amino acid residues, or adding 1-30 amino acid residues, preferably 1-10 amino acid residues, more preferably 1-5 amino acid residues at the N-end or C-end thereof; wherein the obtained amino acid sequence has a sequence identity of $\geq 85\%$ (preferably $\geq 90\%$, more preferably $\geq 95\%$, such as $\geq 96\%$, $\geq 97\%$, $\geq 98\%$, or $\geq 99\%$) with the sequence as shown in positions 261-678 of SEQ ID NO: 1; and the obtained amino acid sequence has the same or similar function as the sequence in (i).

In another preferred embodiment, the structure of the chimeric antigen receptor is shown in the following Formula I:

$$L\text{-}EB\text{-}H\text{-}TM\text{-}C\text{-}CD3\zeta\text{-}RP \qquad (I)$$

wherein,

each "-" is independently a linking peptide or peptide bond;
L is absent or a signal peptide sequence;
EB is an extracellular binding domain;
H is absent or a hinge region;
TM is a transmembrane domain;
C is absent or a co-stimulatory signaling molecule;
CD3$\zeta$ is a cytoplasmic signal transduction sequence derived from CD3$\zeta$;
RP is absent or a reporter protein.

In another preferred embodiment, the reporter protein RP further comprises a self splicing recognition site located at the N-terminus, preferably a T2A sequence.

In another preferred embodiment, the reporter protein RP is a fluorescent protein.

In another preferred embodiment, the reporter protein RP is a mKate2 red fluorescent protein.

In another preferred embodiment, the amino acid sequence of the mKate2 red fluorescent protein is shown in SEQ ID NO: 2.

In another preferred embodiment, L is a signal peptide of a protein selected from a group consisting of CD8, CD28, GM-CSF, CD4, CD137 and a combination thereof.

In another preferred embodiment, L is a signal peptide derived from CD8.

In another preferred embodiment, the amino acid sequence of L is shown in SEQ ID NO: 3.

In another preferred embodiment, H is a hinge region of a protein selected from a group consisting of CD8, CD28, CD137 and a combination thereof.

In another preferred embodiment, H is a hinge region derived from CD8.

In another preferred embodiment, the amino acid sequence of H is shown in SEQ ID NO: 4.

In another preferred embodiment, TM is a transmembrane region of a protein selected from a group consisting of CD28, CD3 epsilon, CD45, CD4, CD5, CD8, CD9, CD16, CD22, CD33, CD37, CD64, CD80, CD86, CD134, CD137, CD154 and a combination thereof.

In another preferred embodiment, TM is a transmembrane region derived from CD28.

In another preferred embodiment, the amino acid sequence of TM is shown in SEQ ID NO: 5.

In another preferred embodiment, C is a co-stimulatory signaling molecule of a protein selected from a group consisting of OX40, CD2, CD7, CD27, CD28, CD30, CD40, CD70, CD134, 4-1BB (CD137), PD1, Dap10, CDS, ICAM-1, LFA-1 (CD11a/CD18), ICOS (CD278), NKG2D, GITR, TLR2 and a combination thereof.

In another preferred embodiment, C is a co-stimulatory signaling molecule derived from 4-1BB.

In another preferred embodiment, the amino acid sequence of C is shown in SEQ ID NO: 6.

In another preferred embodiment, the amino acid sequence of the cytoplasmic signal transduction sequence derived from CD3$\zeta$ is shown in SEQ ID NO: 7.

In another preferred embodiment, the amino acid sequence of the chimeric antigen receptor (CAR) is shown in SEQ ID NO: 8.

In the second aspect of the present invention, it provides a nucleic acid molecule encoding the chimeric antigen receptor according to the first aspect of the present invention.

In the third aspect of the present invention, it provides a vector which contains the nucleic acid molecule according to the second aspect of the present invention.

In another preferred embodiment, the vector is selected from a group consisting of DNA, RNA, plasmid, lentiviral vector, adenoviral vector, retroviral vector, transposon and a combination thereof.

In another preferred embodiment, the vector is a lentiviral vector.

In another preferred embodiment, the vector is selected from a group consisting of pTomo lentiviral vector, plenti, pLVTH, pLJM1, pHCMV, pLBS.CAG, pHR, pLV, etc..

In another preferred embodiment, the vector is a pTomo lentiviral vector.

In another preferred embodiment, the vector further comprises an element selected from a group consisting of: a promoter, a transcription enhancing element WPRE, a long terminal repeat sequence LTR, etc.

In another preferred embodiment, the vector comprises an nucleotide sequece shown in SEQ ID NO: 9.

In the fourth aspect of the present invention, it provides a host cell comprising the vector according to the third aspect of the present invention, or having the exogenous nucleic acid molecule according to the second aspect of the present invention integrated into the chromosome, or expressing the chimeric antigen receptor according to the first aspect of the present invention.

In the fifth aspect of the present invention, it provides an engineered immune cell comprising the vector according to the third aspect of the present invention, or having the exogenous nucleic acid molecule according to the second aspect of the present invention integrated into the chromosome, or expressing the chimeric antigen receptor according to the first aspect of the present invention.

In another preferred embodiment, the immune cell is selected from a group consisting of: a T cell, NK cell, NKT cell, and macrophage.

In another preferred embodiment, the engineered immune cell is a chimeric antigen receptor T cell (CAR-T cell) or a chimeric antigen receptor NK cell (CAR-NK cell).

In another preferred embodiment, the engineered immune cell is a CAR-T cell.

In the sixth aspect of the present invention, it provides a method for the preparation of the engineered immune cell according to the fifth aspect of the present invention, which comprises a step of: transducing the nucleic acid molecule according to the second aspect of the present invention or the vector according to the third aspect of the present invention into an immune cell, thereby obtaining the engineered immune cell.

In another preferred embodiment, the method further comprises a step of detecting the function and effectiveness of the obtained engineered immue cell.

In the seventh aspect of the present invention, it provides a pharmaceutical composition comprising the chimeric antigen receptor according to the first aspect of the present invention, the nucleic acid molecule according to the second aspect of the present invention, the vector according to the third aspect of the present invention, the host cell according to the fourth aspect of the present invention, and/or the engineered immune cell according to the fifth aspect of the present invention, and a pharmaceutically acceptable carrier, diluent or excipient.

In another preferred embodiment, the formulation is a liquid preparation.

In another preferred embodiment, the dosage form of the formulation is an injection.

In another preferred embodiment, the concentration of the engineered immune cell in the formulation is $1 \times 10^3$-$1 \times 10^8$ cells/ml, and preferably $1 \times 10^4$-$1 \times 10^7$ cells/ml..

In the eighth aspect of the present invention, it provides a use of the chimeric antigen receptor according to the first aspect of the present invention, the nucleic acid molecule according to the second aspect of the present invention, the vector according to the third aspect of the present invention, the host cell according to the fourth aspect of the present invention, and/or the engineered immune cell according to the fifth aspect of the present invention, in the preparation of a medicament or a formulation for preventing and/or treating a disease related to the abnormal expression of GAS6 receptor.

In another preferred embodiment, the GAS6 receptor includes but is not limited to Tyro3, AXL, MERTK and the like.

In another preferred embodiment, the abnormal expression of GAS6 receptor refers to the overexpression of GAS6 receptor.

In another preferred embodiment, the overexpression of GAS6 receptor refers to an expression level of GAS6 receptor that is ≥ 1.5 times that under a normal physiological condition, preferably ≥ 2 times, more preferably ≥ 2.5 times.

In another preferred embodiment, the disease related to abnormal expression of GAS6 includes but is not limited to a tumor, venous thromboembolic disease, systemic lupus erythematosus, chronic kidney failure, preeclampsia, and the like.

In another preferred embodiment, the disease related to abnormal expression of GAS6 receptor includes a disease related to abnormal expression of receptor tyrosine kinase AXL.

In another preferred embodiment, the disease related to abnormal expression of AXL includes a tumor, venous thromboembolic disease, systemic lupus erythematosus, chronic kidney failure, preeclampsia, and the like.

In another preferred embodiment, the disease is a malignant tumor with high expression of AXL.

In another preferred embodiment, the tumor includes a hematological tumor and solid tumor.

In another preferred embodiment, the hematological tumor is selected from a group consisting of: acute myeloid leukemia (AML), multiple myeloma (MM), chronic lymphocytic leukemia (CLL), acute lymphocytic leukemia (ALL), diffuse large B cell lymphoma (DLBCL) and a combination thereof.

In another preferred embodiment, the solid tumor is selected from a group consisting of: pancreatic cancer, breast cancer, gastric cancer, hepatobiliary cancer, colorectal cancer, bladder cancer, non-small cell lung cancer, ovarian cancer and esophageal cancer, glioma, lung cancer, prostate cancer, nasopharyngeal cancer and a combination thereof.

In another preferred embodiment, the tumor is selected from a group consisting of: pancreatic cancer, liver cancer, glioma, gastric cancer, and prostate cancer.

In the ninth aspect of the present invention, it provides a use of the engineered immue cell according to the fifth aspect of the present invention or the pharmaceutical composition according to the seventh aspect of the present invention, in preventing and/or treating a tumor or cancer.

In the tenth aspect of the present invention, it provides a method of treating a disease which comprises: administering an effective amount of the engineered immune cell according to the fifth aspect of the present invention, or the pharmaceutical composition of the seventh aspect of the present invention, to a subject in need thereof.

In another preferred embodiment, the disease is related to abnormal expression of a GAS6 receptor.

In another preferred embodiment, the disease is a cancer or tumor.

In another preferred embodiment, the engineered immune cell or the CAR immune cell comprised in the pharmaceutical composition is a cell derived from the subject (an autologous cell).

In another preferred embodiment, the engineered immune cell or the CAR immune cell comprised in the pharmaceutical composition is a cell derived from a healthy individual (an allogeneic cell).

In another preferred embodiment, the method may be utilized in combination with other treatment methods.

In another preferred embodiment, said other treatment method includes chemotherapy, radiotherapy, targeted therapy and other methods.

It should be understood that, within the scope of the present invention, the technical features specifically described above and below (such as the Examples) can be combined with each other, thereby constituting a new or preferred technical solution which needs not be described one by one.

## DESCRIPTION OF DRAWINGS

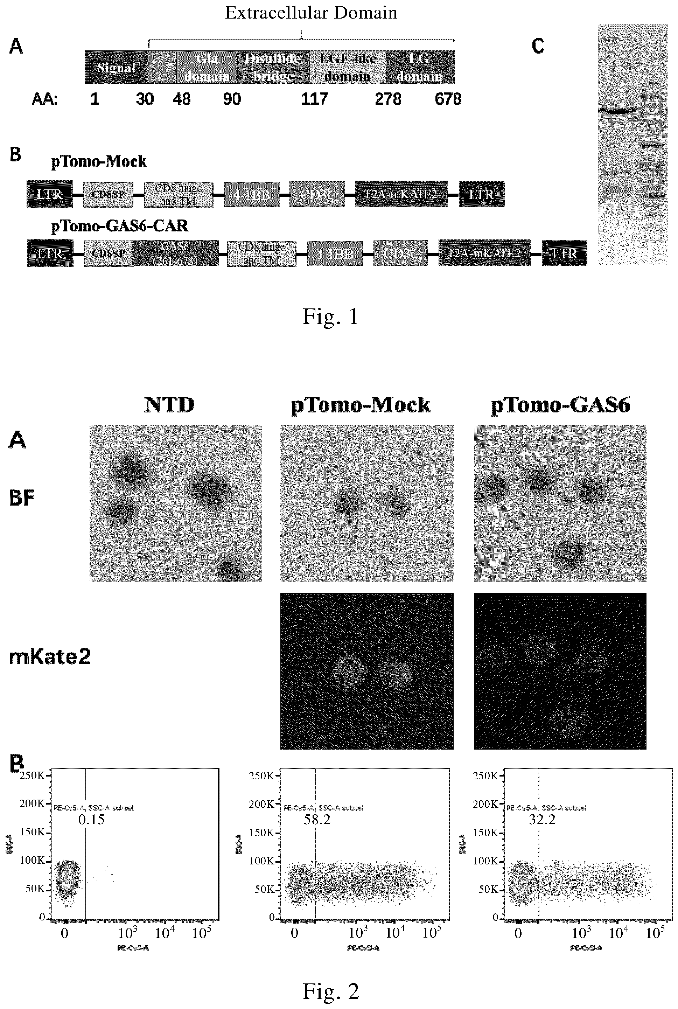

Figure 1 shows a schematic diagram of the construction of GAS6-CAR vector, wherein A shows the schematic diagram of GAS6 sequence, wherein 1-30 AA is the signal peptide, 31-678 AA is the extracellular domain, 49-90 AA is the $\gamma$-carboxyglutamic acid (Gla) domain, 91-117 AA is the disulfide compound bridge, 118-278 AA is the epidermal growth factor (EGF)-like domain, 279-678 AA is the two laminin G-like (LG) domains; B shows the schematic diagram of the control plasmid Mock-CAR and GAS6-CAR, wherein the signal peptide, hinge region, and transmembrane region are derived from human CD8 molecules, 4-1BB is derived from human CD137, CD3$\zeta$ is derived from human CD3 and mKate2 is a fluorescent marker used for detecting CAR expression; and C shows the identification of pTomo-GAS6-CAR vector by HindIII enzyme digestion.

Figure 2 shows the detection result of CAR transfection efficiency, wherein A shows the cell fluorescence expression results of T cells infected with Mock-CAR and GAS6-CAR for 72 hours, where BF represents the bright field and mKate2 is fluorescence expression of CAR; B shows the fluorescence expression result by flow cytometry.

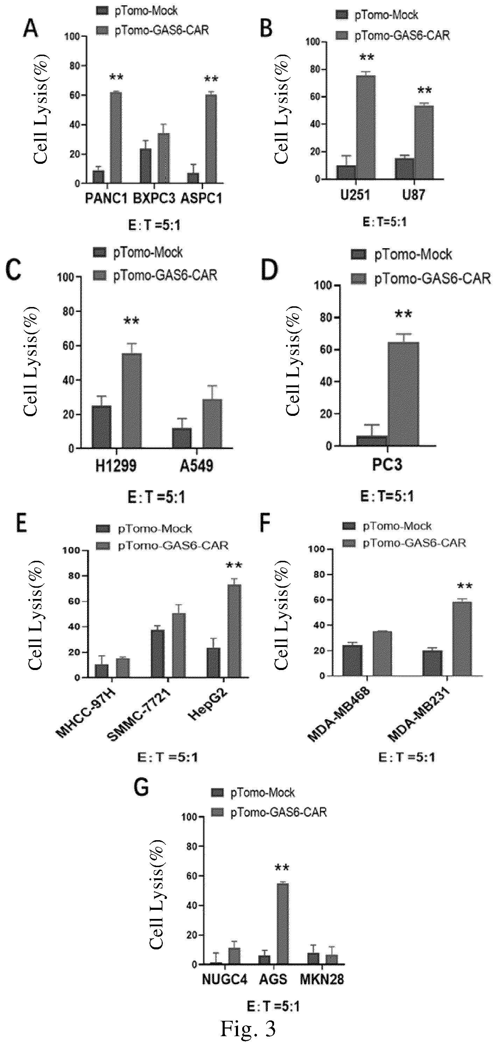

Figure 3 shows the *in vitro* killing effect of GAS6-CAR on different tumor cell lines. A, pancreatic cancer; B, glioma; C, non-small cell lung cancer; D, prostate cancer; E, liver cancer; F, breast cancer; G, gastric caner.

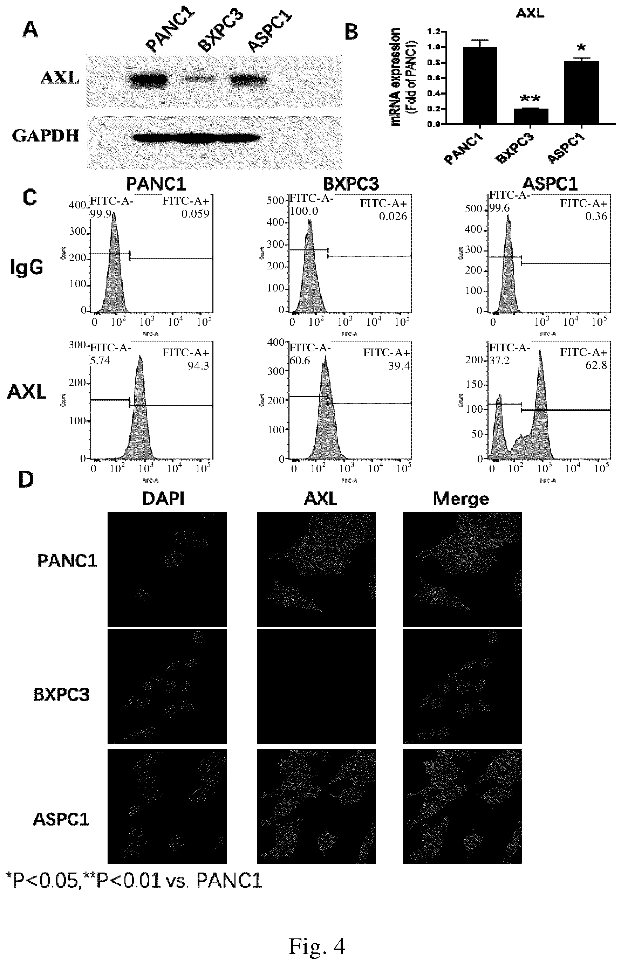

Figure 4 shows the detection result of AXL expression in different pancreatic caner cell lines, wherein A shows the AXL protein expression detected by WB; B shows the AXL mRNA level detected by qPCR; C shows the AXL expression detected by flow cytometry; and D shows the expression level of AXL on the cell membrane detected by immunofluorescence.

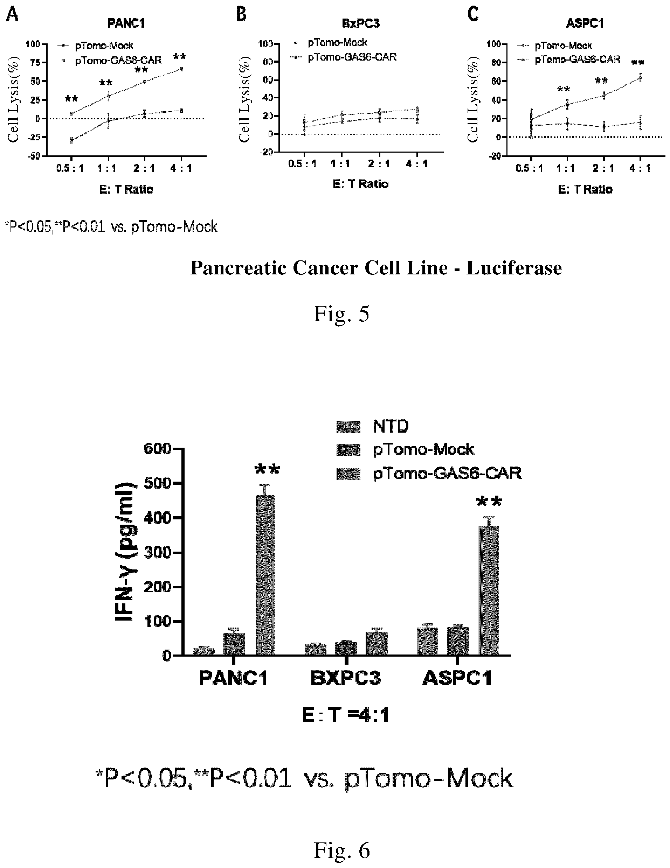

Figure 5 shows the killing effect of gradient GAS6-CAR on different pancreatic cancer cell lines.

Figure 6 shows the IFN$\gamma$ release after GAS6-CAR killing different pancreatic cancer cell lines.

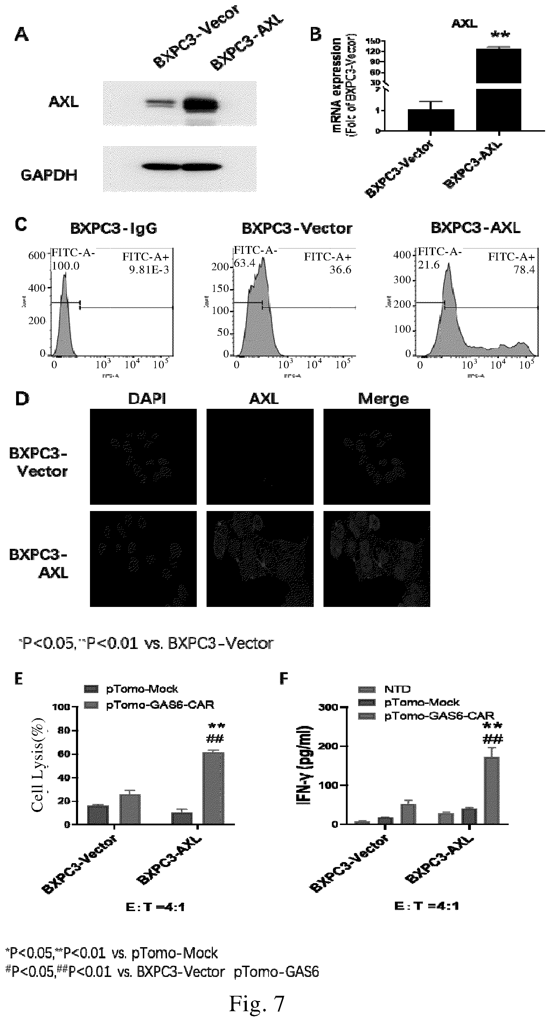

Figure 7 shows the enhanced GAS6-CAR killing effect by overexpression of AXL in the pancreatic cancer cell line BXPC3, wherein A shows the AXL protein expression detected by WB; B shows the AXL mRNA level detected by qPCR; C shows the AXL expression detected by flow cytometry; D shows the expression level of AXL on the cell membrane detected by immunofluorescence; E shows the killing detection of GAS6-CAR against BXPC3-AXL; and F shows the assay of cytokine IFN$\gamma$ release.

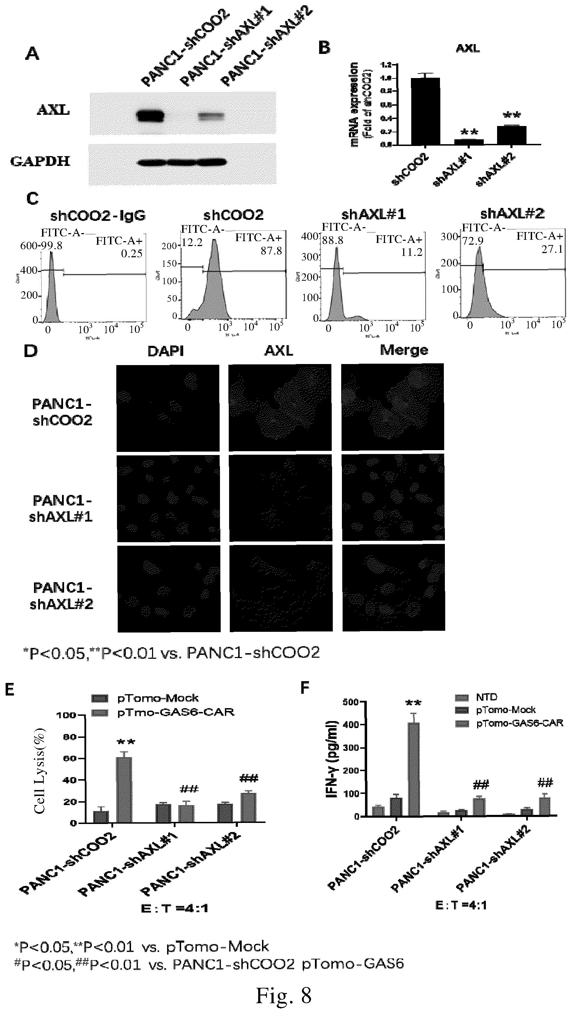

Figure 8 shows the decreased GAS6-CAR killing effect by knockdown of AXL in the pancreatic cancer cell line PANC1, wherein A shows the AXL protein expression detected by WB; B shows the AXL mRNA level detected by qPCR; C shows the AXL expression detected by flow cytometry; D shows the expression level of AXL on the cell membrane detected by immunofluorescence; E shows the killing detection of GAS6-CAR against PANC1-shAXL; and F shows the assay of cytokine IFN$\gamma$ release.

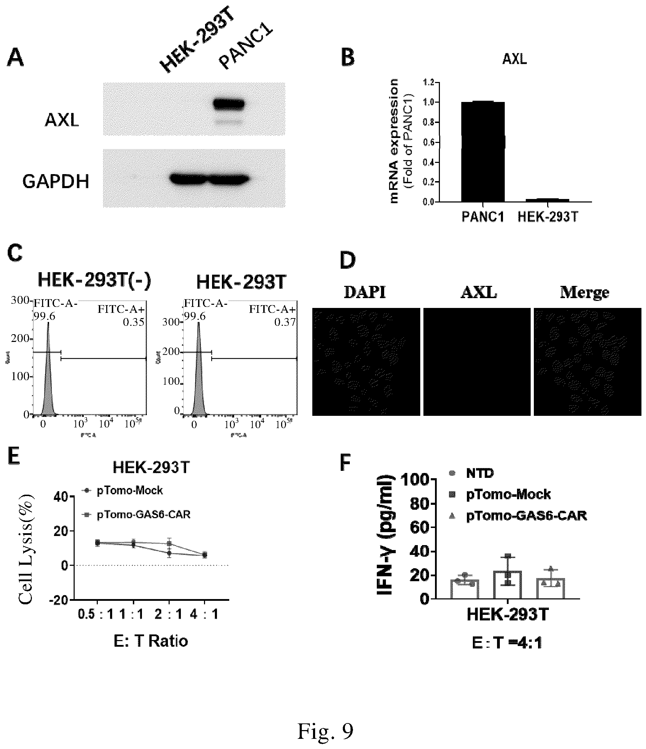

Figure 9 shows the killing effect of GAS6-CAR on normal cell line HEK-293T, wherein A shows the AXL protein expression detected by WB; B shows the AXL mRNA level detected by qPCR; C shows the AXL expression detected by flow cytometry; D shows the expression level of AXL on the cell membrane detected by immunofluorescence; E shows the killing detection of GAS6-CAR against HEK-293T; and F shows the assay of cytokine IFNγ release.

**EMBODIMENTS**

After extensive and intensive research and widely screening, the inventor has developed the preparation and application of a chimeric antigen receptor immune cell based on GAS6 for the first time. The experimental results show that the CAR-T targeting GAS6 receptor of the present invention has a significant killing effect on target cells and a specific anti-tumor cell effect. On this basis, the present invention has been completed.

**Terms**

To make it easier to understand the present invention, certain technical and scientific terms are specifically defined below. Unless otherwise clearly defined herein, all other technical and scientific terms used herein have meanings commonly understood by those skilled in the art in the field to which the present invention belongs. Before describing the present invention, it should be understood that the present invention is not limited to the specific methods and experimental conditions described, as such methods and conditions can be changed. It should also be understood that the terms used herein are only intended to describe the specific embodiments and are not intended to be restrictive. The scope of the present invention will be limited only by the accompanying claims.

Unless otherwise defined, all technical and scientific terms used herein have the same meanings as those commonly understood by those skilled in the art to which the present invention belongs. As used herein, when used in reference to specific enumerated values, the term "about" means that the value may vary by no more than 1% from the enumerated value. For example, as used herein, "about 100" includes all values between 99 and 101 (e.g., 99.1, 99.2, 99.3, 99.4, etc.).

In addition, an positive integer of any one of X1 to X2 represents any positive integer between X1 and X2 (including endpoints), e.g. a positive integer of 1 to 5 includes 1, 2, 3, 4, and 5, and so on.

As used herein, the term "optional" or "optionally" means that the event or situation described thereafter may occur, but not necessarily.

As used herein, the terms "containing" or "comprising (including)" can be open, semi-closed, and closed. In other words, the term also includes "consisting essentially of," or "consisting of."

"Transduction", "transfection", "transformation" or the terms used herein refer to the process of delivering exogenous polynucleotides to host cells, and transcribing and translating to produce peptide products, including the use of plasmid molecules to introduce exogenous polynucleotides into host cells (such as E. coli).

"Gene expression" or "expression" refers to the process of gene transcription, translation, and post-translational modification to produce RNA or protein products of genes.

"Polynucleotide" refers to the polymerized forms of nucleotides of any length, including deoxyribonucleotides (DNA), ribonucleotides (RNA), and heterozygous sequences and analogues thereof. Polynucleotides can include modified nucleotides, such as methylated or capped nucleotides or nucleotide analogues. The term polynucleotides used herein refers to interchangeable single and double stranded molecules. Unless otherwise specified, polynucleotides in any embodiment described herein include double stranded form and two complementary single strands known or predictable to form a double stranded form.

The substitution of conservative amino acids is known in this field. In some embodiments, potential substituted amino acids are within one or more of the following groups: glycine, alanine; and valine, isoleucine, leucine, and proline; aspartate, glutamic acid; asparagine, glutamine; serine, threonine, lysine, arginine, and histidine; and/or phenylalanine, tryptophan, and tyrosine; methionine and cysteine. In addition, the present invention also provides non-conservative amino acid substitutions that allow amino acids from different functional groups to be substituted.

Those skilled in the art will easily understand the meaning of all parameters, sizes, materials, and configurations described herein. The actual parameters, sizes, materials, and configurations depend on the specific application described herein. Those skilled in the art can understand that embodiments or claims are only provided by example, and within the scope of equivalents or claims, the scope that embodiments of the present invention can cover is not limited to the specific description and claims.

The definitions and all definitions used herein should be understood as exceeding those defined in the dictionary or the documents incorporated by reference.

All references, patents, and patent applications herein of the present invention are incorporated by reference to the subject matter cited, and in some cases may include the entire document.

It should be understood that for any method described in this article that includes more than one step, the order of steps is not necessarily limited to the order described in these embodiments.

To make the disclosure easier to understand, some terms are firstly defined. As used in this application, unless expressly stated otherwise herein, each of the following terms shall have the meanings given below. Other definitions are set forth throughout the application.

The term "about" may refer to a value or composition within an acceptable error range for a particular value or composition as determined by those skilled in the art, which will depend in part on how the value or composition is measured or determined.

The term "administering" refers to the physical introduction of a product of the present invention into a subject using any one of various methods and delivery systems known to those skilled in the art, including intravenous, intramuscular, subcutaneous, intraperitoneal, spinal or other parenteral administration, such as by injection or infusion.

TAM Family

The TAM family is a receptor tyrosine kinase (RTK) family that includes members AXL, Tyro3, and MERTK, which play an important role in tumor genesis and development. The AXL gene is located on the long arm of chromosome 19 and consists of 20 exons. It was initially discovered in patients with chronic myeloid leukemia. The protein is encoded by the AXL gene (or UFO or ARK or Tyro7 or JTK11). The full-length AXL protein contains 894 amino acids and has a molecular weight of 98 kDa. However, due to post-translational glycosylation, the actual AXL protein is 120 kDa (partially glycosylated) or 140 kDa (fully glycosylated).

AXL consists of an extracellular domain, a transmembrane domain, and an intracellular domain. The extracellular domain is composed of two immunoglobulin (IgL)-like repeat sequences and two fibronectin type III (Fro III)-like repeat sequences. The Fro III domain is responsible for binding AXL to its ligand GAS6. The intracellular domain of AXL plays an important role in autophosphorylation and downstream kinase activity.

Studies have shown that AXL, including protein and RNA, is highly expressed in various clinical tumor tissues (such as lung cancer, pancreatic cancer, glioma, prostate cancer, breast cancer, liver cancer, colon cancer, ovarian cancer, gastric cancer, etc.) as well as in corresponding tumor cell lines. Additionally, studies have indicated that AXL is highly expressed in macrophages associated with inflammatory diseases and the tumor microenvironment. The high expression of AXL is closely related to the survival, proliferation, metastasis, invasion and epithelial-mesenchymal transition of tumor cells, and sternness of cancer cells. It is also associated with prognosis and survival rates of patients. The expression of AXL even affects the tumor micro environment to promote tumor immunosuppression. Reducing AXL expression using gene knockdown methods can significantly inhibit tumor cell invasion and metastasis, as well as improve prognosis.

In the field of tumor immunotherapy, T cells can exert anti-tumor effects through AXL (such as in triple-negative breast cancer). Compared to AXL-negative tumor cells, AXL-high tumor cells are more susceptible to killing by AXL-CAR T cells. These findings indicate that AXL is an important target molecule for tumor treatment.

## Growth arrest-specific protein 6 (GAS6 or AXSF, AXLLG)

GAS6 is a vitamin K-dependent multi-domain protein with a molecular weight of 75 kDa. It consists of 678 amino acids, including an N-terminal γ-carboxyglutamic acid (Gla) domain (amino acids 49-90) that gives GAS6 the ability to bind to anionic phospholipids on the cell surface and is associated with the stimulation of cell proliferation; four epidermal growth factor (EGF)-like domains (amino acids 118-278); and two laminin G-like (LG) domains (amino acids 279-678). The crystal complex of AXL and GAS6 shows that the C-terminal LG domain of GAS6 binds to the extracellular domains IgL-1 and IgL-2 of AXL.

The GAS6 gene is highly expressed in various cancers and has been considered a marker for poor prognostic. Elevated protein level of GAS6 is also associated with various disease states, including venous thromboembolism, systemic lupus erythematosus, chronic kidney failure, preeclampsia and the like.

GAS6 is a ligand for the TAM family (including Tyro3, AXL and MERTK) and can bind to other members of the TAM family besides AXL. Among them, GAS6 has the highest binding affinity for AXL, and GAS6 is currently the only activating ligand discovered for AXL. When AXL binds to its ligand GAS6 with high affinity, the intracellular kinase domain of the AXL receptor undergoes homodimerization and transphosphorylation, which then recruits adaptor molecules and Src homology 2 (SH2)-containing effector proteins or other phosphotyrosine binding domains (PTB), thus activating downstream signaling pathways such as RAS, RAF, MAPK, ERK1/2, PI3K, TGF-β1/2 and others to exert their functions. The GAS6/AXL signaling pathway is closely related to tumor cell growth, metastasis, invasion, epithelial-mesenchymal transition (EMT), angiogenesis, drug resistance, immune regulation and stem cell maintenance.

Based on this, in the present invention, specific GAS6 fragments are integrated into CAR vectors through genetic engineering for the first time, and used to modify relevant immune cells to achieve cell specific killing of GAS6-binding protein positive cells, which can be used for the treatment of related diseases.

**Chimeric antigen receptor (CAR) of the present invention**

Chimeric antigen receptor (CAR) is composed of extracellular antigen recognition region, transmembrane region, and intracellular co-stimulatory signal region.

The design of CARs has gone through the following process. The first generation CAR has only one intracellular signal component, CD3ζ or FcγRI molecule. Because there is only one activation domain in the cell, it can only cause transient T cell proliferation and less cytokine secretion, and does not provide long-term T cell proliferation signals and sustained antitumor effects in vivo. Therefore, it has not achieved very good clinical efficacy. In the second generation CAR, based on the original structure, a co-stimulatory molecule such as CD28, 4-1BB, OX40 and ICOS is introduced. Compared with the first generation CAR, the function has been greatly improved, and the sustainability of CAR-T cells and the ability to kill tumor cells are further enhanced. Based on the second generation CAR, some new immune stimulatory molecules such as CD27 and CD134 are linked in tandem to develop the third and fourth generation CARs.

The extracellular segment of CAR can recognize a specific antigen, then the signal is transduced through the intracellular domain to induce cell activation, proliferation, cytotoxicity, and secretion of cytokines, thereby clearing target cells. Firstly, the patient's autologous cells (or cells from heterologous donors) are isolated, activated and genetically modified to produce CAR immune cells, and then injected into the same patient's body. This method has an extremely low probability of developing graft-versus-host disease, and the antigen is recognized by immune cells in a non-MHC restricted manner.

CAR immunotherapy has achieved a very high clinical response rate in the treatment of hematological malignancies, which is unmatched by any previous treatment method and has sparked a wave of clinical research worldwide.

Specifically, the chimeric antigen receptor (CAR) of the present invention comprises an extracellular domain, a transmembrane domain, and an intracellular domain.

The extracellular domain comprises a target-specific binding element. The extracellular domain can be an ScFv based on antigen-antibody specific binding, or a natural sequence based on ligand-receptor specific binding or a derivative thereof.

In the present invention, the extracellular domain of the chimeric antigen receptor is a GAS6 protein or fragment thereof that can specifically bind to the AXL target of the CAR of the present invention. More preferably, the extracellular binding domain of the chimeric antigen receptor of the present invention has an amino acid sequence of positions 261-678 of the sequence shown in SEQ ID NO: 1.

The intracellular domain includes a co-stimulatory signaling region and a ζ chain. The co-stimulatory signaling region refers to a part of the intracellular domain that includes a co-stimulatory molecule. The co-stimulatory molecule is a cell surface molecule required for efficient response of lymphocytes to antigens, rather than a antigen receptor or its ligand.

A linker (or linking peptide) can be incorporated between the extracellular domain and the transmembrane domain of the CAR, or between the cytoplasmic domain and the transmembrane domain of the CAR. As used herein, the term "linker" generally refers to any oligopeptide or polypeptide that plays a role of linking the transmembrane domain to the extracellular domain or the cytoplasmic domain in a polypeptide chain. The linker may comprise 0-300 amino acids, preferably 2-100 amino acids and most preferably 3-50 amino acids.

When the CAR of the present invention is expressed in T cell, antigen recognition can be performed based on antigen binding specificity. When the CAR binds to its associated antigen, it affects tumor cell, causing tumor cell to fail to grow, to death or to be affected otherwise, causing the patient's tumor burden to shrink or eliminate. The antigen binding domain is preferably fused to one or more intracellular domains of the co-stimulatory molecule and the ζ chain. Preferably, the antigen binding domain is fused with a CD8 signaling domain, and an intracellular domain of a CD3ζ signaling domain.

In the present invention, the extracellular domain of the CAR of the present invention also includes conservative variants thereof based on the sequence, which means that compared with the amino acid sequence of positions 261-678 (or positions 279-678) of SEQ ID NO: 1, there are at most 10, preferably at most 8, more preferably at most 5 amino acids replaced by amino acids with the same or similar properties to form a polypeptide.

In the present invention, the number of added, deleted, modified, and/or substituted amino acids is preferably not more than 40% of the total number of amino acids in the initial amino acid sequence, more preferably not more than 35%, more preferably 1-33%, more preferably 5-30%, more preferably 10-25% and more preferably 15-20%.

In he present invention, the number of added, deleted, modified and/or substituted amino acids is typically 1, 2, 3, 4, or 5, preferably 1-3, preferably 1-2, and preferably 1.

As for the hinge region and the transmembrane region (transmembrane domain), the CAR can be designed to comprise a transmembrane domain fused to the extracellular domain of the CAR. In one embodiment, a transmembrane domain that is naturally associated with one of the domains in the CAR is used. In some embodiments, transmembrane domains may be selected or modified by amino acid substitutions to avoid binding such domains to the transmembrane domain of the same or different surface membrane proteins, thereby minimizing the interaction with other members of the receptor complexes.

The intracellular domain in the CAR of the present invention comprises the 4-1BB co-stimulatory domain and the signaling domain of CD3ζ.

In one embodiment of the present invention, the CAR can specifically target AXL.

**Chimeric antigen receptor immune cell (CAR-immune cell)**

In the present invention, provided is a chimeric antigen receptor immune cell comprising a chimeric antigen receptor of the present invention that specifically targets a GAS6 receptor (preferably AXL).

The chimeric antigen receptor immune cell of the present invention can be a CAR-T cell, a CAR-NK cell, or a CAR-macrophage. Preferably, the chimeric antigen receptor immune cell of the present invention is a CAR-T cell.

As used herein, the terms "CAR-T cell", "CAR-T", and "CAR-T cell of the present invention" all refer to the CAR-T cell described in the fifth aspect of the present invention.

CAR-T cells have the following advantages over other T cell-based therapies: (1) the action process of CAR-T cells is not limited by MHC; (2) given that many tumor cells express the same tumor markers, a CAR gene targeting a particular tumor marker, once being completely constructed, can be widely utilized; (3) CARs can utilize both tumor protein markers and non-protein markers of carbohydrates and lipids, expanding the target range of tumor markers; (4) the use of patient autologous cells reduces the risk of rejection reactions; (5) CAR-T cells have immune memory function and can survive in the body for a long time.

As used herein, the terms "CAR-NK cell", "CAR-NK", and "CAR-NK cell of the present invention" all refer to the CAR-NK cell described in the fifth aspect of the present invention. The CAR-NK cell of the present invention can be used in tumors with high expression of GAS6 receptors (preferably AXL).

Natural killer (NK) cells are a major type of immune effector cells that protect the body from viral infections and tumor cell invasion through non antigen-specific pathways. Engineered (genetic modified) NK cells may acquire new functions, including the ability to specifically recognize tumor antigens and enhanced anti-tumor cytotoxicity.

Compared with CAR-T cells, CAR-NK cells also have advantages such as: (1) directly killing tumor cells by releasing perforin and granzyme, without killing normal cells in the body; (2) releasing a small amount of cytokines, thereby reducing the risk of cytokine storms; (3) extremely easiness to be expanded and developed into "ready-made" products *in vitro.* In addition, it is similar to CAR-T cell therapy.

As used herein, the terms "CAR-macrophage(M) cell", "CAR-macrophage (M)", and "CAR-M cell of the present invention" all refer to the CAR-macrophage described in the fifth aspect of the present invention. The CAR-macrophage of the present invention can be used in treating tumors with high expression of GAS6 receptors (preferably AXL).

Macrophages are phagocytic cells derived from monocytes and play an important role in the body's innate and cellular immunity. Engineered (genetic modified) NK cells may acquire enhanced ability to specifically recognize tumor antigens and enhanced anti-tumor cytotoxicity.

**Vector**

The nucleic acid sequences coding for the desired molecules can be obtained using recombinant methods known in the art, such as, for example by screening libraries from cells expressing the gene, by deriving the gene from a vector known to include the same, or by isolating directly from cells and tissues containing the same, using standard techniques. Alternatively, the gene of interest can be produced synthetically.

The present invention also provides vectors comprising the nucleic acid molecule of the present invention. Vectors derived from retroviruses such as the lentivirus are suitable tools to achieve long-term gene transfer since they allow long-term, stable integration of a transgene and its propagation in daughter cells. Lentiviral vectors have the advantage over vectors derived from onco-retroviruses such as murine leukemia viruses in that they can transduce non-proliferating cells, such as hepatocytes. They also have the advantage of low immunogenicity.

In brief, the expression cassette or nucleic acid sequence of the present invention is typically and operably linked to a promoter, and incorporated into an expression vector. The vectors can be suitable for replication and integration in eukaryotes. Typical cloning vectors contain transcription and translation terminators, initiation sequences, and promoters useful for regulation of the expression of the desired nucleic acid sequence.

The expression constructs of the present invention may also be used for nucleic acid immune and gene therapy, using standard gene delivery protocols. Methods for gene delivery are known in the art. See, e.g., U.S, Pat. Nos. 5,399,346, 5,580,859, and 5,589,466, which are incorporated by reference herein in entirety. In another embodiment, the present invention provides a gene therapy vector.

The nucleic acid can be cloned into various vectors. For example, the nucleic acid can be cloned into a vector including, but not limited to a plasmid, a phagemid, a phage derivative, an animal virus, and a cosmid. Vectors of particular interest include expression vectors, replication vectors, probe generation vectors, and sequencing vectors.

Further, the expression vector may be provided to a cell in the form of a viral vector. Viral vector technology is well known in the art and is described, for example, in Sambrook et al (2001 , Molecular Cloning: A Laboratory Manual, Cold Spring Harbor Laboratory, New York), and in other virology and molecular biology manuals. Viruses which are useful as vectors include but are not limited to, retroviruses, adenoviruses, adeno-associated viruses, herpes viruses, and lentiviruses. In general, a suitable vector contains an origin of replication functional in at least one organism, a promoter sequence, convenient restriction endonuclease sites, and one or more selectable markers, (e.g., WO 01/96584; WO 01/29058; and U.S, Pat. No. 6,326, 193).

A number of viral based systems have been developed for transferring a gene into mammalian cells. For example, retroviruses provide a convenient platform for gene delivery systems. A selected gene can be inserted into a vector and packaged in retroviral particles using techniques known in the art. The recombinant virus can then be isolated and delivered to cells of the subject either in vivo or ex vivo. A number of retroviral systems are known in the art. In some embodiments, adenovirus vectors are used. A number of adenovirus vectors are known in the art. In one embodiment, lentivirus vectors are used.

Additional promoter elements, e.g., enhancers, regulate the frequency of transcriptional initiation. Typically, these are located in the region 30-110 bp upstream of the start site, although a number of promoters have recently been shown to contain functional elements downstream of the start site as well. The spacing between promoter elements frequently is flexible, so that promoter function is preserved when elements are inverted or moved relative to one another. In the thymidine kinase (tk) promoter, the spacing between promoter elements can be increased to 50 bp apart before activity begins to decline. Depending on the promoter, it appears that individual elements can function either cooperatively or independently to activate transcription.

One example of a suitable promoter is the immediate early cytomegalovirus (CMV) promoter sequence. This promoter sequence is a strong constitutive promoter sequence capable of driving high levels of expression of any polynucleotide sequence operatively linked thereto. Another example of a suitable promoter is Elongation Growth Factor-1α (EF-1α). However, other constitutive promoter sequences may also be used, including, but not limited to the simian virus 40 (SV40) early promoter, mouse mammary tumor virus (MMTV), human immunodeficiency virus (HIV) long terminal repeat (LTR) promoter, MoMuLV promoter, an avian leukemia virus promoter, an Epstein-Barr virus immediate early promoter, a Rous sarcoma virus promoter, as well as human gene promoters such as, but not limited to, the actin promoter, the myosin promoter, the hemoglobin promoter, and the creatine kinase promoter. Further, the present invention should not be limited to the use of constitutive promoters. Inducible promoters are also contemplated as part of the present invention. The use of an inducible promoter provides a molecular switch capable of turning on expression of the polynucleotide sequence which it is operatively linked when such expression is desired, or turning off the expression when expression is not desired. Examples of inducible promoters include, but are not limited to a metallothionein promoter, a glucocorticoid promoter, a progesterone promoter, and a tetracycline promoter.

In order to assess the expression of a CAR polypeptide or portions thereof, the expression vector to be introduced into a ceil can also contain either a selectable marker gene or a reporter gene or both to facilitate identification and selection of expressing cells from the population of cells sought to be transfected or infected through viral vectors. In other aspects, the selectable marker may be carried on a separate piece of DNA and used in a co-transfection procedure. Both selectable markers and reporter genes may be flanked with appropriate regulatory sequences to enable expression in the host cells. Useful selectable markers include, for example, antibiotic-resistance genes, such as neo and the like.

Reporter genes are used for identifying potentially transfected cells and for evaluating the functionality of regulatory sequences. In general, a reporter gene is a gene that is not present in or expressed by the recipient organism or tissue and that encodes a polypeptide whose expression is manifested by some easily detectable property, e.g., enzymatic activity. Expression of the reporter gene is assayed at a suitable time after the DNA has been introduced into the recipient cells. Suitable reporter genes may include genes encoding luciferase, beta-galactosidase, chloramphenicol acetyl transferase, secreted alkaline phosphatase, or the green fluorescent protein gene (e.g., Ui-Tei et al., 2000 FEBS Letters 479: 79-82). In one embodiment of the present invention, the reporter gene is a gene encoding the mKate2 red fluorescent protein. Suitable expression systems are well known and may be prepared using known techniques or obtained commercially. In general, the construct with the minimal 5' flanking region showing the highest level of expression of reporter gene is identified as the promoter. Such promoter regions may be linked to a reporter gene and used to evaluate agents for the ability to modulate promoter-driven transcription.

Methods of introducing and expressing genes into a cell are known in the art. In the context of an expression vector, the vector can be readily introduced into a host cell, e.g., mammalian, bacterial, yeast, or insect cell by any method in the art. For example, the expression vector can be transferred into a host cell by physical, chemical, or biological means.

Physical methods for introducing a polynucleotide into a host cell include calcium phosphate precipitation, lipofection, particle bombardment, microinjection, electroporation, and the like. Methods for producing cells comprising vectors and/or exogenous nucleic acids are well-known in the art. See, for example, Sambrook et al. (2001, Molecular Cloning: A Laboratory Manual, Cold Spring Harbor Laboratory, New York). A preferred method for the introduction of a polynucleotide into a host cell is calcium phosphate transfection.

Biological methods for introducing a polynucleotide of interest into a host cell include the use of DNA and RNA vectors. Viral vectors, and especially retroviral vectors, have become the most widely used method for inserting genes into mammalian, e.g., human cells. Other viral vectors can be derived from lentivirus, poxviruses, herpes simplex virus I, adenoviruses and adeno-associated viruses, and the like. See, for example, U.S. Pat, Nos. 5,350,674 and 5,585,362.

Chemical means for introducing a polynucleotide into a host cell include colloidal dispersion systems, such as macromolecule complexes, nanocapsules, microspheres, beads, and lipid-based systems including oil-in-water emulsions, micelles, mixed micelles, and liposomes. An exemplary colloidal system for use as a delivery vehicle in vitro and in vivo is a liposome (e.g., an artificial membrane vesicle).

In the case where a non-viral delivery system is utilized, an exemplary delivery vehicle is a liposome. The use of lipid formulations is contemplated for the introduction of the nucleic acids into a host cell (in vitro, ex vivo or in vivo). In another aspect, the nucleic acid may be associated with a lipid. The nucleic acid associated with a lipid may be encapsulated in the aqueous interior of a liposome, interspersed within the lipid bilayer of a liposome, attached to a liposome via a linking molecule that is associated with both the liposome and the oligonucleotide, entrapped in a liposome, complexed with a liposome, dispersed in a solution containing a lipid, mixed with a lipid, combined with a lipid, contained as a suspension in a lipid, contained or complexed with a micelle, or otherwise associated with a lipid. Lipid, lipid/DNA or lipid/expression vector associated compositions are not limited to any particular structure in solution. For example, they may be present in a bilayer structure, as micelles, or with a "collapsed" structure. They may also simply be interspersed in a solution, possibly forming aggregates that are not uniform in size or shape. Lipids are fatty substances which may be naturally occurring or synthetic lipids. For example, lipids include the fatty droplets that naturally occur in the cytoplasm as well as the class of compounds which contain long-chain aliphatic hydrocarbons and their derivatives, such as fatty acids, alcohols, amines, amino alcohols, and aldehydes.

In a preferred embodiment of the present invention, the vector is a lentiviral vector.

## Formulation

The present invention provides a formulation (or preparation) comprising the chimeric antigen receptor (CAR) according to the first aspect of the present invention, the nucleic acid molecule according to the second aspect of the present invention, the vector according to the third aspect of the present invention, or the host cell according to the fourth aspect of the present invention or the engineered immune cell according to the fifth aspect of the present invention, and a pharmaceutically acceptable carrier, diluent or excipient. In one embodiment, the formulation is a liquid preparation. Preferably, the formulation is an injection. Preferably, the concentration of the CAR-T cells in the formulation is $1 \times 10^3$-$1 \times 10^8$ cells/ml, more preferably $1 \times 10^4$-$1 \times 10^7$ cells/ml.

In one embodiment, the formulation may comprises buffers such as neutral buffered saline, phosphate buffered saline and the like; carbohydrates such as glucose, mannose, sucrose or dextrans, mannitol; proteins; polypeptides or amino acids such as glycine; antioxidants; chelating agents such as EDTA or glutathione; adjuvants (e.g., aluminum hydroxide); and preservatives. The formulation of the present invention is preferably formulated for intravenous administration.

## Therapeutic application

The present invention comprises therapeutic applications by using cells (e.g., T cells) transduced with a lentiviral vector (LV) encoding the expression cassette of the present invention. The transduced T cells can target the tumor cell marker, receptor tyrosine kinase (preferably AXL), synergistically activate immun cells, and cause T cell immune responses, thereby significantly increasing the killing efficiency against tumor cells.

Therefore, the present invention also provides a method for stimulating a T cell-mediated immune response to a target cell population or tissue in a mammal comprising a step of administering to the mammal a CAR-T cell of the present invention.

In one embodiment, the present invention comprises a cell therapy, wherein T cells from autologous patient (or heterologous donor) are isolated, activated and genetically modified to generate CAR-T cells, and then injected into the same patient. The probability of graft-versus-host-disease in the way is extremely low, and antigens are recognized by T cells in a non-MHC-restricted manner. In addition, one CAR-T can treat all cancers that express the antigen. Unlike antibody therapy, CAR-cells are able to replicate in vivo and result in long-term persistence that can lead to sustained tumor control.

In one embodiment, the CAR-T cells of the present invention can undergo robust in vivo T cell expansion and can persist for an extended period. In addition, the CAR mediated immune response may be part of an adoptive immunotherapy approach in which CAR-modified T cells induce an immune response specific to the antigen binding moiety in the CAR. For example, CAR-T cell targeting a receptor tyrosine kinase (preferably AXL) elicits an immune response specific against cells expressing receptor tyrosine kinase (preferably AXL).

Although the data disclosed herein specifically disclose lentiviral vector comprising GAS6 protein of a fragment thereof, hinge and transmembrane domains, and 4-1BB and CD3ζ signaling domains, it is contemplated that the present invention include any number of variations for each of the components of the construct as described elsewhere herein.

Cancers that may be treated include tumors that are unvascularized or largely unvascularized, and tumors that are vascularized. Cancers may include non-solid tumors (such as hematological tumors, for example, leukemias and lymphomas) or solid tumors. Types of cancers to be treated with the CARs of the present invention include, but are not limited to, carcinoma, blastoma, and sarcoma, and certain leukemia or lymphoid malignancies, benign and malignant tumors, and malignancies e.g., sarcomas, carcinomas, and melanomas. Adult tumors/cancers and pediatric tumors/cancers are also included.

Hematologic cancers are cancers of the blood or bone marrow. Examples of hematological (or hematogenous) cancers include leukemias, including acute leukemias (such as acute lymphocytic leukemia, acute myelocytic leukemia, acute myelogenous leukemia and myeloblasts, promyeiocytic, myelomonocytic, monocytic and erythroleukemia), chronic leukemias (such as chronic myelocytic (granulocytic) leukemia, chronic myelogenous leukemia, and chronic lymphocytic leukemia), polycythemia vera, lymphoma, Hodgkin's disease, non-Hodgkin's lymphoma (indolent and high grade forms), multiple myeloma, Waldenstrom's macroglobulinemia, heavy chain disease, myelodysplastic syndrome, hairy cell leukemia and myelodysplasia.

Solid tumors are abnormal masses of tissue that usually do not contain cysts or liquid areas. Solid tumors can be benign or malignant. Different types of solid tumors are named for the type of cells that form them (such as sarcomas, carcinomas, and lymphomas). Examples of solid tumors (such as sarcomas and carcinomas) include fibrosarcoma, myxosarcoma, liposarcoma, mesothelioma, malignant lymphoma, pancreatic cancer, ovarian cancer, breast cancer, gastric cancer, hepatobiliary cancer, colorectal cancer, bladder cancer, non-small cell lung cancer, ovarian and esophageal cancer, glioblastoma, lung cancer, prostate cancer, nasopharyngeal cancer, etc..

The CAR-modified T cells of the present invention may also serve as a type of vaccine for ex vivo immunization and/or in vivo therapy in a mammal. Preferably, the mammal is a human.

With respect to ex vivo immunization, at least one of the following occurs in vitro prior to administering the cells into a mammal: i) expanding the cells, ii) introducing a nucleic acid encoding a CAR to the cells, and/or iii) cryopreservation of the cells.

Ex vivo procedures are well known in the art and are discussed more fully below. Briefly, cells are isolated from a mammal (preferably a human) and genetically modified (i.e., transduced or transfected in vitro) with a vector expressing a CAR disclosed herein. The CAR-modified cell can be administered to a mammalian recipient to provide a therapeutic benefit. The mammalian recipient may be a human and the CAR-modified cell can be autologous with respect to the recipient. Alternatively, the cells can be allogeneic, syngeneic or xenogeneic with respect to the recipient.

In addition to using a cell-based vaccine in terms of ex vivo immunization, the present invention also provides compositions and methods for in vivo immunization to elicit an immune response directed against an antigen in a patient.

The present invention provides a method for treating tumors comprising administering to a subject in need thereof, a therapeutically effective amount of the CAR-modified T cells of the present invention.

The CAR-modified T cells of the present invention may be administered either alone, or as a pharmaceutical composition in combination with diluents and/or with other components such as IL-2, IL-17 or other cytokines or cell populations. Briefly, pharmaceutical compositions of the present invention may comprise a target cell population as described herein, in combination with one or more pharmaceutically or physiologically acceptable carriers, diluents or excipients. Such compositions may comprise buffers such as neutral buffered saline, phosphate buffered saline and the like; carbohydrates such as glucose, mannose, sucrose or dextrans, mannitol; proteins; polypeptides or amino acids such as glycine; antioxidants; chelating agents such as EDTA or glutathione; adjuvants (e.g., aluminum hydroxide); and preservatives. Compositions of the present invention are preferably formulated for intravenous administration.

Pharmaceutical compositions of the present invention may be administered in a manner appropriate to the disease to be treated (or prevented). The quantity and frequency of administration will be determined by such factors as the condition of the patient, and the type and severity of the patient's disease, although appropriate dosages may be determined by clinical trials.

When "an effective amount", "an immunologically effective amount", "an anti-tumor effective amount", "an tumor-inhibiting effective amount", or "a therapeutic amount" is indicated, the precise amount of the compositions of the present invention to be administered can be determined by a physician with consideration of individual differences in age, weight, tumor size, extent of infection or metastasis, and condition of the patient (subject). It can generally be stated that a pharmaceutical composition comprising the T cells described herein may be administered at a dosage of 104 to 109 cells/kg body weight, preferably 105 to 106 cells/kg body weight, including all integer values within those ranges. T cell compositions may also be administered multiple times at these dosages. The cells can be administered by using infusion techniques that are commonly known in immunotherapy (see, e.g., Rosenberg et al,, New Eng. J. of Med. 319: 1676, 1988). The optimal dosage and treatment regime for a particular patient can readily be determined by one skilled in the art of medicine by monitoring the patient for signs of disease and adjusting the treatment accordingly.

The administration of the subject compositions may be carried out in any convenient manner, including by aerosol inhalation, injection, ingestion, transfusion, implantation or transplantation. The compositions described herein may be administered to a patient subcutaneously, intradermaliy, intratumorally, intranodally, intramedullary, intramuscularly, by intravenous (i.v.) injection, or intraperitoneally. In one embodiment, the T cell compositions of the present invention are administered to a patient by intradermal or subcutaneous injection. In another embodiment, the T cell compositions of the present invention are preferably administered by i.v. injection. The compositions of T cells may be injected directly into a tumor, lymph node, or site of infection.

In certain embodiments of the present invention, cells activated and expanded using the methods described herein, or other methods known in the art where T cells are expanded to therapeutic levels, are administered to a patient in conjunction with (e.g., before, simultaneously or following) any number of relevant treatments, including but not limited to treatment with agents such as antiviral therapy, cidofovir and interleukin-2, Cytarabine (also known as ARA-C) or natalizumab treatment for MS patients or efalizumab treatment for psoriasis patients or other treatments for specific tumor patients. In further embodiments, the T cells of the present invention may be used in combination with chemotherapy, radiation, immunosuppressive agents, such as cyclosporin, azathioprine, methotrexate, mycophenolate, and FK506, antibodies, or other immunotherapeutic agents. In a further embodiment, the cell compositions of the present invention are administered to a patient in conjunction with (e.g., before, simultaneously or following) bone marrow transplantation, or the use of chemotherapy agents such as, fludarabine, external-beam radiation therapy (XRT), cyclophosphamide. For example, in one embodiment, subjects may undergo standard treatment with high dose chemotherapy followed by peripheral blood stem cell transplantation. In certain embodiments, following the transplant, subjects receive an infusion of the expanded immune cells of the present invention. In an additional embodiment, expanded cells are administered before or following surgery.

The dosage of the above treatments to be administered to a patient will vary with the precise nature of the condition being treated and the recipient of the treatment. The scaling of dosages for human administration can be performed according to practices in the field. In general, $1 \times 10^6$ to $1 \times 10^{10}$ of CAR-T cells of the present invention can be applied to patients by means of, for example, intravenous infusion in each treatment or each course of treatment.

Main advantages of the present invention include:

1) Target specificity: AXL is not expressed on the cell membrane of normal cells, but highly expressed on tumor tissue cell membranes and macrophages. Therefore, the present CAR only specifically killing tumor cells and macrophages highly expressing AXL on the cell membrane and has no killing effect on normal cells.

2) The present invention utilizes a ligand-receptor binding mechanism instead of the traditional scfv. The conserved nature of receptor-ligand interactions determines that safety tests in animals, especially primates, better reflect their safety in the human body.

3) GAS6 can also bind to other members of the TAM family and avoid immune escape of tumor cells through multi-target effect.

The invention is further illustrated below in conjunction with specific embodiments. It should be understood that the examples are not intended to limit the scope of the invention. The experimental methods in the following examples which do not specify the specific conditions are usually in accordance with conventional conditions, such as conditions described in Sambrook et al., Molecular Cloning: Laboratory Manual (New York: Cold Spring Harbor Laboratory Press, 1989), or in accordance with the conditions recommended by the manufacturer. Unless otherwise stated, percentages and parts are by weight.

unless otherwise described, the reagents, plasmids and cells in the Examples of the present application are commercially available.

**Table 1 Sequence**

| Name of sequence | Sequence | SEQ ID NO |
|---|---|---|
| Full-length GAS6 | MAPSLSPGPAALRRAPQLLLLLLAAECALAALLPARE ATQFLRPRQRRAFQVFEEAKQGHLERECVEELCSREE | 1 |

(continued)

| Name of sequence | Sequence | SEQ ID NO |
|---|---|---|
| protein | AREVFENDPETDYFYPRYLDCINKYGSPYTKNSGFAT CVQNLPDQCTPNPCDRKGTQACQDLMGNFFCLCKAG WGGRLCDKDVNECSQENGGCLQICHNKPGSFHCSCH SGFELSSDGRTCQDIDECADSEACGEARCKNLPGSYS CLCDEGFAYSSQEKACRDVDECLQGRCEQVCVNSPG SYTCHCDGRGGLKLSQDMDTCEDILPCVPFSVAKSVK SLYLGRMFSGTPVIRLRFKRLQPTRLVAEFDFRTFDPE GILLFAGGHQDSTWIVLALRAGRLELQLRYNGVGRVT SSGPVINHGMWQTISVEELARNLVIKVNRDAVMKIAV AGDLFQPERGLYHLNLTVGGIPFHEKDLVQPINPRLD GCMRSWNWLNGEDTTIQETVKVNTRMQCFSVTERGS FYPGSGFAFYSLDYMRTPLDVGTESTWEVEVVAHIRP AADTGVLFALWAPDLRAVPLSVALVDYHSTKKLKKQ LVVLAVEHTALALMEIKVCDGQEHVVTVSLRDGEAT LEVDGTRGQSEVSAAQLQERLAVLERHLRSPVLTFAG GLPDVPVTSAPVTAFYRGCMTLEVNRRLLDLDEAAY KHSDITAHSCPPVEPAAA | |
| mKate2 red fluorescent protein | MSELIKENMHMKLYMEGTVNNHHFKCTSEGEGKPYE GTQTMRIKAVEGGPLPFAFDILATSFMYGSKTFINHTQ GIPDFFKQSFPEGFTWERVTTYEDGGVLTATQDTSLQ DGCLIYNVKIRGVNFPSNGPVMQKKTLGWEASTETLY PADGGLEGRADMALKLVGGGHLICNLKTTYRSKKPA KNLKMPGVYYVDRRLERIKEADKETYVEQHEVAVAR YCDLPSKLGHKLN | 2 |
| Signal peptide | MALPVTALLLPLALLLHAARP | 3 |
| CD8 hinge region | TTTPAPRPPTPAPTIASQPLSLRPEACRPAAGGAVHTR GLDFACD | 4 |
| CD28 transmembr ane region | IYIWAPLAGTCGVLLLSLVITLYC | 5 |
| Co-stimulat ory signaling molecule | KRGRKKLLYIFKQPFMRPVQTTQEEDGCSCRFPEEEE GGCELR | 6 |

(continued)

| Name of sequence | Sequence | SEQ ID NO |
|---|---|---|
| derived from 4-1BB | | |
| Cytoplasmi c signal transduction sequence derived from CD3ζ | VKFSRSADAPAYKQGQNQLYNELNLGRREEYDVLDK RRGRDPEMGGKPRRKNPQEGLYNELQKDKMAEAYS EIGMKGERRRGKGHDGLYQGLSTATKDTYDALHMQ ALPPR | 7 |
| Preferred full-length CAR of the present invention | MALPVTALLLPLALLLHAARPHCDGRGGLKLSQDMD TCEDILPCVPFSVAKSVKSLYLGRMFSGTPVIRLRFKR LQPTRLVAEFDFRTFDPEGILLFAGGHQDSTWIVLALR AGRLELQLRYNGVGRVTSSGPVINHGMWQTISVEELA RNLVIKVNRDAVMKIAVAGDLFQPERGLYHLNLTVG GIPFHEKDLVQPINPRLDGCMRSWNWLNGEDTTIQET VKVNTRMQCFSVTERGSFYPGSGFAFYSLDYMRTPLD VGTESTWEVEVVAHIRPAADTGVLFALWAPDLRAVP LSVALVDYHSTKKLKKQLVVLAVEHTALALMEIKVC DGQEHVVTVSLRDGEATLEVDGTRGQSEVSAAQLQE RLAVLERHLRSPVLTFAGGLPDVPVTSAPVTAFYRGC MTLEVNRRLLDLDEAAYKHSDITAHSCPPVEPAAATT TPAPRPPTPAPTIASQPLSLRPEACRPAAGGAVHTRGL DFACDIYIWAPLAGTCGVLLLSLVITLYCKRGRKKLL YIFKQPFMRPVQTTQEEDGCSCRFPEEEEGGCELRVKF SRSADAPAYKQGQNQLYNELNLGRREEYDVLDKRRG RDPEMGGKPRRKNPQEGLYNELQKDKMAEAYSEIGM KGERRRGKGHDGLYQGLSTATKDTYDALHMQALPPR | 8 |

(continued)

| Name of sequence | Sequence | SEQ ID NO |
|---|---|---|
| Nucleotide sequence encoding the preferred full-length CAR of the present invention | atggccctgcccgtcaccgctctgctgctgcccccttgctctgcttcttcatgcagcaa ggccgcactgtgacgggcgtggggggcctcaagctgtcccaggacatggacacct gtgaggacatcttgccgtgcgtgcccttcagcgtggccaagagtgtgaagtccttgt acctgggccggatgttcagtgggaccccccgtgatccgactgcgcttcaagaggctg cagcccaccaggctggtagctgagtttgacttccggacctttgaccccgagggcat cctcctctttgccggaggccaccaggacagcacctggatcgtgctggccctgaga gccggccggctggagctgcagctgcgctacaacggtgtcggccgtgtcaccagc agcggcccggtcatcaaccatggcatgtggcagacaatctctgttgaggagctggc gcggaatctggtcatcaaggtcaacagggatgctgtcatgaaaatcgcggtggccg | 9 |

(continued)

| Name of sequence | Sequence | SEQ ID NO |
|---|---|---|
|  | gggacttgttccaaccggagcgaggactgtatcatctgaacctgaccgtgggaggt attcccttccatgagaaggacctcgtgcagcctataaaccctcgtctggatggctgc atgaggagctggaactggctgaacggagaagacaccaccatccaggaaacggtg aaagtgaacacgaggatgcagtgcttctcggtgacggagagaggctctttctaccc cgggagcggcttcgccttctacagcctggactacatgcggacccctctggacgtcg ggactgaatcaacctgggaagtagaagtcgtggctcacatccgcccagccgcaga cacaggcgtgctgtttgcgctctgggcccccgacctccgtgccgtgcctctctctgt ggcactggtagactatcactccacgaagaaactcaagaagcagctggtggtcctgg ccgtggagcatacggccttggccctaatggagatcaaggtctgcgacggccaaga gcacgtggtcaccgtctcgctgagggacggtgaggccaccctggaggtggacgg caccagggggccagagcgaggtgagcgccgcgcagctgcaggagaggctggcc gtgctcgagaggcacctgcggagccccgtgctcacctttgctggcggcctgccag atgtgccggtgacttcagcgccagtcaccgcgttctaccgcggctgcatgacactg gaggtcaaccggaggctgctggacctggacgaggcggcgtacaagcacagcga catcacggcccactcctgcccccccgtggagcccgccgcagccaccacgacgcc agcgccgcgaccaccaacaccggcgcccaccatcgcTAGCcagcccctgtcc ctgcgcccagaggcgtgccggccagcggcgggggggcgcagtgcacacgaggg ggctggacttcgcctgtgatatctacatctgggcgcccttggccgggacttgtggggg tccttctcctgtcactggttatcacccttactgcaaacggggcagaaagaaactcct gtatatattcaaacaaccatttatgagaccagtacaaactactcaagaggaagatgg ctgtagctgccgatttccagaagaagaagaaggaggatgtgaactgagagtgaagt tcagcaggagcgcagacgcccccgcgtacaagcagggccagaaccagctctata acgagctcaatctaggacgaagagaggagtacgatgttttggacaagagacgtgg ccgggaccctgagatggggggaaagccgagaaggaagaaccctcaggaaggc ctgtacaatgaactgcagaaagataagatggcggaggcctacagtgagattgggat gaaaggcgagcgccggagggggcaaggggcacgatggcctttaccagggtctca gtacagccaccaaggacacctacgacgcccttcacatgcaggccctgcccctcg c |  |

Table 2 Cell Lines

| Cell Lines | Type |
| --- | --- |
| PANC 1 | Pancreatic cancer cell |
| BXPC3 | Pancreatic cancer cell |
| ASPC1 | Pancreatic cancer cell |
| U251 | Glioma cell |
| U87 | Glioma cell |
| H1299 | Non-small cell lung cancer cell |
| A549 | Non-small cell lung cancer cell |
| PC3 | Prostate cancer cell |
| MHCC-97H | Liver cancer cell |
| HepG2 | Liver cancer cell |
| SMMC-7721 | Liver cancer cell |
| MDA-MB468 | Breast cancer cell |
| MDA-MB231 | Breast cancer cell |
| NUGC4 | Gastric cancer cell |
| MKN28 | Gastric cancer cell |
| AGS | Gastric cancer cell |

**Example 1: Preparation of GAS-CAR Vector**

According to the nucleotide sequence of GAS6 (NM 000820.4) and gene sequence information of human CD8 signal peptide, human CD8$\alpha$ hinge region, human CD8 transmembrane region, human 4-1BB intracellular region and human CD3$\zeta$ intracellular region, the corresponding nucleotide sequences were obtained by synthetic methods or PCR methods. CD8 signal peptide and GAS6 extracellular region (18 amino acids of epidermal growth factor-like domain were retained at the N-terminus of GAS6 to ensure the integrity of GAS6 extracellular domain cohesin G-like structure) were synthesized. The nucleotide sequence of the CAR molecule was digested with AgeI (Thermo) and NheI (Thermo), ligated by T4 DNA ligase (NEB) and inserted into the lentiviral vector pTomo, which had already been integrated with the sequences of CD8 transmembrane region, 4-1BB costimulatory domain and CD3$\zeta$ signaling region. The vector was transformed into competent E. coli (Stbl3).

The recombinant plasmid was sequenced, and the sequencing results were aligned to confirm the correctness of the plasmid. Universal sequencing primers were used. Both sequencing and digestion identification shows that the coding sequence of the CAR was correctly inserted into the predetermined position of the plasmid (Fig. 1C).

All plasmids were extracted using the endotoxin-free large-scale extraction kit from QIAGEN. The purified plasmids were used to transfect HEK-293T cells with Beyotime lipo6000 for lentivirus packaging.

**Example 2: Packaging Virus**

HEK-293T cells were performed in a 15 cm culture dish for virus packaging. Transfection was carried out when the confluence of HEK-293T cells was about 80%-90%. A plasmid mixture dissolved in 2ml OPTIMEM was prepared (core plasmid 20ug, pCMV$\Delta$R8.9 10ug, PMD2.G 4ug); and 2ml OPTIMEM and 68ul of lipo 6000 were combined in another tube. After set at room temperature for 5 minutes, the plasmid complex was added to the liposome complex and set at room temperature for 20 minutes. The mixture was then added dropwise to the HEK-293T cells and incubated at 37°C for 6 hours before removing the medium. Pre-warmed complete media were added. Viral supernatants were collected at 48 hours and 72 hours post-transfection, centrifuged at 3000 rpm for 20 minutes at 4°C, filtered through a 0.45um filter membrane, and then concentrated by centrifugation at 25000rpm for 2.5 hours at 4°C. The concentrated virus was dissolved overnight in 30 ul of virus dissolving solution, and the virus titer was detected by QPCR. The results shows that the viral titer fulfills the requirement.

**Example 3: Preparation of CAR-T cells**

Mononuclear cells were isolated from human peripheral blood using Ficoll separation solution, and purified CD3+ T cells were obtained using RosetteSep Human T Cell Enrichment Cocktail (Stemcell technologies). T cells were activated with CD3/CD28 magnetic beads (Life technology) and stimulated and cultured with 200U/ml of IL2 (PeproTech) added for 48 hours before viral infection. Lentivirus was used to infected T cells at an MOI=100 in the presence of lentiboost to prepare CAR-T cells. The medium was replaced after one-day infection.

**Example 4: Detection of the Positive Rate of CAR-T Cells Infection by Flow Cytometry**

CAR-T cells and NTD cells (control group) were centrifuged and collected separately after 72 hours of virus infection. They were then washed once with PBS, and the supernatant was discarded. The cells were resuspended in PBS containing 2% FBS, and the positive rate was detected by flow cytometry.
The results of the transfection efficiency are shown in Fig. 2.
As shown in Fig. 2A, the cleavage of CAR-T2A-mKate2 fusion protein expressed by CAR-T cells forms a mKate2 protein which exhibits red fluorescence in the cell.
Fig. 2B shows that the positive expression rate of CAR or mKate2CAR-T is approximately 30%, assayed by flow cytometry.

**Example 5: Detection of AXL Expression in Various Target Cells**

(1)Cell immunofluorescence: Target cells were seeded onto round slips of a 24-well plate. After 24 hours, cells were fixed with 4% paraformaldehyde (PFA) for 20 min, and washed three times in PBST for 5 min each. They were then blocked with 10% goat serum for 1 h at room temperature and incubated at four degrees overnight with antibodies that specifically recognize AXL. The following day, the cells were washed three times with PBST for 5 minutes each. Cells were added with a secondary antibody that specific recognizes mHSP70 and is labeled with CY3, and incubated at room temperature for 1 hour. The cells were washed three times with PBS, and the nuclei were stained with DAPI. Confocal microscopy imaging was performed.
(2) Flow cytometry: 1 million cells were collected, fixed with 4% PFA at room temperature for 15min and centrifugal washed by 1X PBS. Cells were than permeabilized with 100% methanol on ice for 15 min and centrifugal washed by 1X PBS. After this, cells were resuspended with 100 $\mu$l of diluted primary antibody (1:300) and incubated for 1 hour at room temperature, and centrifugal washed by 1X PBS. The supernatant was discarded. The operation was repeated. Cells were resuspended with 100 $\mu$l of diluted fluorescent-conjugated secondary antibody (FITC-488 anti-rabbit), incubated for 30 min at room temperature in the dark, and centrifugal washed in 1X PBS. The supernatant was discarded. The operation was repeated. The cells were resuspended with 300 $\mu$L of 1X PBS, and then subjected to the flow cytometry.
(3) Western blot: Cells were collected from a 6-cm culture dish and centrifuged at 5500 r/min for 5 minutes at 4°C. The supernatant were discarded, then RIPA cell lysis buffer containing protease inhibitor PMSF was added based on the cell count. The cells were then lysed on ice for 20 minutes and centrifuged at 14,000 r/min for 30 minutes at 4°C. The supernatant was collected for concentration measurement. Protein samples of 50$\mu$g were loaded for protein electrophoresis to detect the AXL expression of target cell.
(4) qPCR: Cells were collected from a 6-cm culture dish. The culture medium was removed. Then 1 ml of Trizol was added to lyse the cells, and the mixture was left to stand at room temperature for 5 minutes. 200 $\mu$l of chloroform per 1ml of Trizol was added, inverted 6-8 times for mixing, and left to stand at room temperature for 5 minutes. The mixture was then centrifuged at 12000g for 15 minutes at 4°C. The clear supernatant was transferred to another centrifuge tube. An equal volume of isopropanol was added, inverted for mixing, and left to stand at room temperature for 10 minutes. After centrifuging at 12000g for 10 minutes at 4°C, the supernatant was discarded. 1 ml of 70% ethanol (prepared with RNase-free H2O) was added for washing, and centrifuged at 7500g for 5 minutes at room temperature. The supernatant was discarded, and the mixture was left to stand for 10 minutes at room temperature to dry the RNA. 30 ul of RNase-free water was added to dissolve the RNA. The concentration of RNA was measured using Nandrop 2000, and the integrity and quantitative accuracy of RNA were detected using 1% agarose gel electrophoresis. CDNA was synthesized using the RevertAidTM First Strand cDNA Synthesis Kit (Thermo Scientific) according to the manufacturer's instructions. mRNA levels were detected.

The detection results of AXL expression in various cell lines are shown in Fig. 4. The expression of AXL in target cells was detected by Western blotting (Fig. 4A), qPCR (Fig. 4B), and flow cytometry (Fig. 4C). The results consistently show that PANC1 and ASPC1 highly express AXL, while BXPC3 have low AXL expression. Further verification through immunofluorescence localization confirms that AXL is highly expressed on the cell membranes of PANC1 and ASPC1, as well as on the cell membranes of PANC1 and BXPC3 (Fig. 4D).

**Example 6: Construction of Luciferase-Expressing Target Cells**

The lentiviral packaging steps for pTomo-CMV-Luciferase-IRES-Puro are identical to those in Example 2.

PANC1, BXPC3, ASPC1, U251, U87, H1299, A549, PC3, MHCC-97H, HepG2, SMMC-7721, MDA-MB468, MDA-MB231, NUGC4AGS, MKN28, and HEK-293T cells were infected with the virus. Puromycin (1ug/ml) was used for screening for 2 weeks. Then PANC1, BXPC3, ASPC1, U251, U87, H1299, A549, PC3, MHCC-97H, HepG2, SMMC-7721, MDA-MB468, MDA-MB231, NUGC4AGS, MKN28-luciferase, and HEK-293T-luciferase cells were successfully obtained.

**Example 7: Killing Effect of CART Cells**

In this example, the killing ability of the CAR-T cells of the present invention against different target cells was tested. The target cells used include: target cells with high AXL expression: PANC1, ASPC1, U251, U87, H1299, PC3, HepG2, MDA-MB231, AGS; target cells that do not express or express low levels of AXL: BXPC3, A549, MHCC-97H, SMMC-7721, MDA-MB468, NUGC4, MKN28.

PANC1, BXPC3, ASPC1, U251, U87, H1299, A549, PC3, MHCC-97H, HepG2, SMMC-7721, MDA-MB468, MDA-MB231, NUGC4AGS, and MKN28-luciferase cells were digested, counted, and adjusted to a cell density of $2\times10^4$/ml. 100ul of luciferase cells were seeded into a 96-well plate. CAR-T and NT cells were adjusted to a density of $1\times10^5$ cells/ml. They were then seeded into the black 96-well plate at an E:T ratio of 5:1, in a volume of 100 ul per well. The above target cells and T cells were mixed and incubated in a culture incubator for 24 hours.

The PANC1, BXPC3, ASPC1-luciferase cells were counted and adjusted to a density of $2\times10^4$ cells/ml. 100ul of PANC1, BXPC3, ASPC1-luciferase cells were seeded into a 96-well plate. CAR-T and NT cells were adjusted to a density of $1\times10^5$ cells/ml. They were then seeded into a black 96-well plate at E:T ratios of 0.5:1, 1:1, 2:1, and 4:1, in a volume of 100 ul per well. The above target cells and T cells were mixed and incubated in a culture incubator for 24 hours.

The supernatant was collected and stored at -80°C for the detection of IFNγ release (see Example 8). Cell killing was detected using the Promega fluorescence detection kit. First, the cells were lysed with 20 ul of 1*PLB lysis buffer for 20 minutes. Then, 100 ul of substrate was added to each well, and the reaction was immediately detected using a BioTek plate reader.

The cytotoxicity killing rate % = (1 - the fluorescence value of target cells with effector cells / the fluorescence value of target cells without effector cells) × 100%

The killing effects of GAS6-CAR on different tumor cell lines are shown in Fig. 3, pancreatic cancer cells (A); glioma cells (B); non-small cell lung cancer cells (C); prostate cancer cells (D); liver cancer cells (E); breast tumor cells (F); gastric cancer cells (G). The results show that GAS6-CAR has good killing effect on multiple cancer cell lines.

The killing effects of gradient GAS6-CAR on different pancreatic cancer cell lines are shown in Fig. 5. (A) Killing effect of gradient GAS6-CAR on PANC1; (B) killing effect of gradient GAS6-CAR on BXPC3; (C) killing effect of gradient GAS6-CAR on ASPC1. The results indicate that GAS6 CAR-T cells have a gradually increasing killing effect on tumor cells highly expressing AXL as the effector:target (E:T) ratio increases.

**Example 8: Detection of IFNγ Cytokine Release**

In this example, the release of cytokines under the co-incubation of CAR-T cells of the present invention and target cells is detected. The cell supernatant of the co-incubate from cell killing assay was used for detection.

The method is as follows: the supernatant of the CAR-T cells of the present invention co-incubated with PANC1, BXPC3, ASPC1, HEK-293T target cells (E:T ratio of 4:1) in Example 7 was taken for detection of IFNγ according to the IFN gamma Human ELISA Kit (life technology).

The standard samples were dissolved using Standard Dilution Buffer, and grandiently diluted into standard samples of 1000 pg/ml, 500 pg/ml, 250 pg/ml, 125 pg/ml, 62.5 pg/ml, 31.2 pg/ml, 15.6 pg/ml, and 0 pg/ml.

To each well, 50 ul Incubation buffer, 50 ul sample to be detected and 50 ul IFNγ biotin conjugate solution were added, mixed well and incubated at room temperature for 90 minutes.

Then the following steps are performed in order:

(1) Wash the wells four times with 1* Wash Buffer, leaving each time for one minute.
(2) Add 100 ul 1* Streptavidin-HRP solution to each well and incubate at room temperature for 45 minutes.
(3) Wash the wells four times with 1* Wash Buffer, leaving each time for one minute.
(4) Add 100 ul Stabilized chromogen and incubate at room temperature for 30 minutes.
(5) Add 100 ul Stop solution to each well and mix well.
(6) Detect the absorbance at 450 nm.

The results are shown in Fig. 6. The cytokines secreted by GAS6-CAR-T killing PANC1 and ASPC1 increased significantly, but that for BXPC3 does not change significantly. The results show that the killing effect of GAS6-CAR-T cells on tumor cells is accompanied by the release of IFNy, suggesting that the killing effect is related to the release of IFN$\gamma$.

**Example 9: Effect of Overexpression of AXL on GAS-CART Tumor Killing**

Based on the CDS sequence of AXL, the AXL overexpression plasmid (EX-Z7835-Lv105-B, ORF lentiviral expression clone) was purchased from Yijin Biotechnology for transient transfection.

The day before transfection, 50 thousand target cells were seeded in each well of a six-well plate for cultivation. Before transfection, the medium in each well of the six-well plate was replaced by 2 ml of fresh culture medium (containing serum, antibiotics-free). Two clean and sterile centrifuge tubes was added with 125 $\mu$l of Opti-MEM® Medium to each. Then one tube was added with 2.5 $\mu$g of plasmid DNA, which was gently mixed with a pipette. The other tube was added with 5 $\mu$l of Lipo6000™ transfection reagent, which was gently mixed with a pipette. After standing at room temperature for 5 minutes, the DNA-containing culture medium was gently added to the culture medium containing Lipo6000™ transfection reagent using a pipette. The centrifuge tube was gently inverted or gently mixed with a pipette, and left to stand at room temperature for another 5 minutes before adding it to a 6-well plate and mixing. Overexpression efficiency was detected after 48 hours, and GAS6-CAR-T killing detection was performed.

Various pancreatic cancer cells BXPC3-Vector and BXPC3-AXL-luciferase were digested, counted and adjusted to a cell density of $2\times10^4$/ml. 100ul of luciferase cells were seeded into a 96-well plate. CAR-T and NT cells were adjusted to a density of $1 \times 10^5$ cells/ml. They were then seeded into the black 96-well plate at an E:T ratio of 4:1, in a volume of 100 ul per well. The above target cells and T cells were mixed and incubated in a culture incubator for 24 hours.

The killing effect of GAS6-CAR-T on the pancreatic cancer cell line BXPC3 which overexpresses AXL is shown in Fig. 7. Fig. 7-A shows the AXL protein expression detected by WB. Fig. 7-B shows the AXL mRNA level detected by qPCR. Fig. 7-C shows the AXL expression detected by flow cytometry. Fig. 7-D shows the expression level of AXL on the cell membrane detected by immunofluorescence. Figure 7-E shows the killing effect of GAS6-CAR on BXPC3 overexpressing AXL. Figure 7-F shows the IFN$\gamma$ release of GAS6-CAR-T against BXPC3 overexpressing AXL. The results show that compared with the BXPC3-Vector group, the killing rate and IFN$\gamma$ release of GAS6-CAR-T cells against BXPC3-AXL cells overexpressing AXL were significantly increased. These results indicat that GAS6-CAR-T cells had a significantly enhanced killing effect on AXL-overexpressing tumor cells.

Example 10: Effect of Knock-down of AXL on GAS-CAR-T Killing Effect

According to the shRNA sequence library provided by Sigma, the MISSION shRNA Lentiviral Transduction Particles were used, the validated shRNA in the CDS region was selected, and each shRNA selected was subjected to BLAST on the NCBI to ensure the specificity of the target.

The day before transfection, 100 thousand HEK-293T cells were seeded in each well of a 6-cm plate for cultivation. Before transfection, the medium in each well of the 6-cm plate plate was replaced by 5ml of fresh culture medium (containing serum, antibiotics-free). Two clean and sterile centrifuge tubes was added with 250 $\mu$l of Opti-MEM® Medium to each. Then one tube was added with 5 $\mu$g of plasmid DNA, which was gently mixed with a pipette. The other tube was added with 10 $\mu$l of Lipo6000™ transfection reagent, which was gently mixed with a pipette. After standing at room temperature for 5 minutes, the DNA-containing culture medium was gently added to the culture medium containing Lipo6000™ transfection reagent using a pipette. The centrifuge tube was gently inverted or gently mixed with a pipette, and left to stand at room temperature for 20 minutes before adding it to a 6-cm plate and mixing. The supernatant was collected after 48 h and 72 h,respectively, then filtered and centrifuged at 3000 r/min for 15 min at 4°C to obtain the virus-containing supernatant.

The day before transfection, 50 thousand target cells were seeded in each well of a six-well plate for cultivation. Before transfection, the medium in each well of the six-well plate was replaced with 1 ml of fresh culture medium (containing serum, antibiotics-free). Then 1 ml of viral supernatant and 2 ul polybrane (10 mg/ml) were added. After 24 h, the medium was replaced with complete medium, and the shRNA knockdown efficiency and GAS6-CAR-T killing effect was detected after 72 h. *In vitro* killing experiment was carried out as described previously. The killing effect of GAS6-CAR on PANC1 and PANC1-shAXL was detected by measuring changes of fluorescence intensity.

PANC1-shCOO2, PANC1-shAXL#1, PANC1-shAXL#2-luciferase cells were digested and counted, and adjusted to a cell density of $2\times10^4$/ml. 100ul of luciferase cells were seeded into a 96-well plate. CAR-T and NT cells were adjusted to a density of $1\times10^5$ cells/ml. They were then seeded into the black 96-well plate at an E:T ratio of 4:1, in a volume of 100 ul per well. The above target cells and T cells were mixed and incubated in a culture incubator for 24 hours, then subjected to the killing effect detection.

The results are shown in Fig. 8. Fig. 8-A shows the AXL protein expression detected by WB. Fig. 8-B shows the AXL mRNA level detected by qPCR. Fig. 8-C shows the AXL expression detected by flow cytometry. Fig. 8-D shows the expression level of AXL on the cell membrane detected by immunofluorescence. Figure 8-E shows the killing effect of GAS6-CAR on PANC1 after AXL knockdown. Figure 8-F shows the IFN$\gamma$ release from the killing of GAS6-CAR against PANC1 after AXL silencing. The results show that, GAS6-CAR-T cells exhibit significantly lower killing rate and IFN$\gamma$ release for PANC1-shAXL#1 and PANC1-shAXL#1 cells with AXL knockdown compared with those for the control PANC1-shCOO2 cells. The results show that the knockdown of AXL on the cell membrane reduces the killing effect of GAS6-CAR-T.

### Example 11: Killing Effect of GAS6-CAR-T on Non-tumor Cells

HEK-293T cells, a human embryonic kidney cell line, were seeded into a black 96-well plate at E:T ratios of 0.5:1, 1:1, 2:1 and 4:1. GAS6-CAR-T cells were co-incubated with HEK-293T-luciferase cells, and the killing of HEK-293T cells by GAS6-CAR-T was detected by the change of fluorescence value.

The results are shown in Fig. 9. Fig. 9-A shows the AXL protein expression detected by WB. Fig. 9-B shows the AXL mRNA level detected by qPCR. Fig. 9-C shows the AXL expression detected by flow cytometry. Fig. 9-D shows the expression level of AXL on the cell membrane detected by immunofluorescence. Figure 9-E shows the killing effect of GAS6-CAR on HEK-293T. Figure 9-F shows the IFN$\gamma$ release from the killing of GAS6-CAR against HEK-293T.

The results indicate that GAS6-CART has no significant killing effect on the non-tumor HEK-293T cells.

All documents mentioned in the present invention are incorporated by reference herein as if each document were incorporated separately by reference. Furthermore, it should be understood that after reading the foregoing teachings of the invention, various changes or modifications may be made to the invention by those skilled in the art and that these equivalents are equally within the scope of the claims appended to this application.

### Claims

1. A chimeric antigen receptor (CAR) comprising an extracellular binding domain which comprises a structure based on a GAS6 protein comprising an amino acid sequence set forth in SEQ ID NO: 1 or a fragment thereof and is able to specifically bind to a GAS6 receptor.

2. The chimeric antigen receptor according to claim 1, wherein the extracellular binding domain comprises a GAS6 protein or a fragment thereof which has an amino acid sequence as shown in SEQ ID NO: 1 or has an amino acid sequence as shown in positions 1 to 678 (preferably positions 31-678, more preferably positions 118-678, and more preferably positions 261-678) of SEQ ID NO: 1.

3. The chimeric antigen receptor according to claim 1, wherein the amino acid sequence of the GAS6 protein or the fragment thereof is selected from a group consisting of:

   (i) a sequence as shown in positions 261-678 of the sequence of SEQ ID NO: 1; and
   (ii) on the basis of the sequence shown in positions 261-678 of the sequence of SEQ ID NO: 1, an amino acid sequence obtained by replacing, deleting, altering or inserting one or more amino acid residues, or adding 1-30 amino acid residues, preferably 1-10 amino acid residues, more preferably 1-5 amino acid residues at the N-end or C-end thereof; wherein the obtained amino acid sequence has a sequence identity of $\geq 85\%$ (preferably $\geq 90\%$, more preferably $\geq 95\%$, such as $\geq 96\%$, $> 97\%$, $> 98\%$, or $\geq 99\%$) with the sequence as shown in positions 261-678 of SEQ ID NO: 1; and the obtained amino acid sequence has the same or similar function as the sequence in (i).

4. The chimeric antigen receptor according to any one of claims 1-3, wherein the structure of the chimeric antigen receptor is shown in the following Formula I:

$$L\text{-}EB\text{-}H\text{-}TM\text{-}C\text{-}CD3\zeta\text{-}RP \qquad (I)$$

wherein,

  each "-" is independently a linking peptide or peptide bond;
  L is absent or a signal peptide sequence;
  EB is an extracellular binding domain;
  H is absent or a hinge region;
  TM is a transmembrane domain;
  C is absent or a co-stimulatory signaling molecule;
  CD3ζ is a cytoplasmic signal transduction sequence derived from CD3ζ;
  RP is absent or a reporter protein.

5. The chimeric antigen receptor according to claim 4, wherein the amino acid sequence of the chimeric antigen receptor is shown in SEQ ID NO: 8.

6. A nucleic acid molecule encoding the chimeric antigen receptor according to claim 1.

7. A vector comprising the nucleic acid molecule according to claim 6.

8. A host cell comprising the vector according to claim 7, or having the exogenous nucleic acid molecule according to claim 6 integrated into the chromosome thereof, or expressing the chimeric antigen receptor according to claim 1.

9. An engineered immune cell comprising the vector according to claim 7, or having the exogenous nucleic acid molecule according to claim 6 integrated into the chromosome thereof, or expressing the chimeric antigen receptor according to claim 1.

10. A method for the preparation of the engineered immune cell according to claim 9, which comprises a step of transducing the nucleic acid molecule according to claim 6 or the vector according to claim 7 into the immune cell, thereby obtaining the engineered immune cell.

11. A pharmaceutical composition comprising the chimeric antigen receptor according to claim 1, the nucleic acid molecule according to claim 6, the vector according to claim 7, the host cell according to claim 8, and/or the engineered immune cell according to claim 9, and a pharmaceutically acceptable carrier, diluent or excipient.

12. Use of the chimeric antigen receptor according to claim 1, the nucleic acid molecule according to claim 6, the vector according to claim 7, or the host cell according to claim 8, and/or the engineered immune cell according to claim 9, in the preparation of a medicament or a formulation for preventing and/or treating a disease related to the abnormal expression of GAS6 receptor.

13. The use according to claim 12, wherein the disease related to abnormal expression of GAS6 receptor comprises a drug or formulation for a disease related to abnormal expression of a receptor tyrosine kinase AXL.

14. The use according to claim 13, wherein the disease related to abnormal expression of AXL comprises a tumor, venous thromboembolic disease, systemic lupus erythematosus, chronic kidney failure, preeclampsia, and the like.

15. The use according to claim 14, wherein the tumor is selected from a group consisting of: pancreatic cancer, liver cancer, glioma, gastric cancer, and prostate cancer.

Fig. 1

Fig. 2

Fig. 3

*P<0.05,**P<0.01 vs. PANC1

Fig. 4

*P<0.05,**P<0.01 vs. pTomo-Mock

**Pancreatic Cancer Cell Line - Luciferase**

Fig. 5

*P<0.05,**P<0.01 vs. pTomo-Mock

Fig. 6

*P<0.05,**P<0.01 vs. BXPC3-Vector

*P<0.05,**P<0.01 vs. pTomo-Mock
#P<0.05,##P<0.01 vs. BXPC3-Vector pTomo-GAS6

Fig. 7

*P<0.05,**P<0.01 vs. PANC1-shCOO2

*P<0.05,**P<0.01 vs. pTomo-Mock
#P<0.05,##P<0.01 vs. PANC1-shCOO2 pTomo-GAS6

Fig. 8

Fig. 9

**INTERNATIONAL SEARCH REPORT**

| International application No. |
| --- |
| **PCT/CN2022/130701** |

| A. | CLASSIFICATION OF SUBJECT MATTER |
| --- | --- |

C07K 14/705(2006.01)i;  C12N 15/10(2006.01)i;  C12N 15/63(2006.01)i;  A61P 35/00(2006.01)i;  A61P 17/00(2006.01)i; A61P 9/10(2006.01)i;  A61P 13/12(2006.01)i;  A61P 15/00(2006.01)i

According to International Patent Classification (IPC) or to both national classification and IPC

| B. | FIELDS SEARCHED |
| --- | --- |

Minimum documentation searched (classification system followed by classification symbols)

C07K C12N A61P

Documentation searched other than minimum documentation to the extent that such documents are included in the fields searched

Electronic data base consulted during the international search (name of data base and, where practicable, search terms used)

WPABSC; CNTXT; DWPI; ENTXTC; VEN; PubMed; Web of Science; CNKI; 万方, WANFANG: 嵌合抗原受体, CAR, 配体, AXL, GAS6. 中国专利生物序列检索系统, China Patent Biological Sequence Search System; Genbank; EMBL; STN和检索的序列, STN and retrieved sequences: SEQ ID NO: 1, 8 et al.

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| Y | CN 110177808 A (F1 ONCOLOGY, INC.) 27 August 2019 (2019-08-27) <br> claims 1-5, and description, paragraphs [0065]-[0089] | 1-15 |
| Y | CN 111629734 A (UNIVERSITY OF SOUTHERN CALIFORNIA) 04 September 2020 (2020-09-04) <br> claims 1-47, and description, paragraphs [0007]-[0017] | 1-15 |
| A | CN 110483639 A (FUDAN UNIVERSITY et al.) 22 November 2019 (2019-11-22) <br> description, paragraphs [0013]-[0166] | 1-15 |
| A | CN 112426438 A (SHANGHAI SINOBAY BIO-TECH CO., LTD.) 02 March 2021 (2021-03-02) <br> description, paragraphs [0007]-[0044] | 1-15 |
| A | Xiao H, et al. "growth arrest-specific protein 6 precursor [Homo sapiens], NP_000811.1" <br> *GenBank,* 31 October 2021 (2021-10-31), <br> entire document, in particular an amino acid sequence | 1-15 |

☐ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **11 January 2023** | **20 January 2023** |

| Name and mailing address of the ISA/CN | Authorized officer |
| --- | --- |
| **China National Intellectual Property Administration (ISA/ CN)** <br> **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

| International application No. |
| --- |
| **PCT/CN2022/130701** |

| Box No. I       Nucleotide and/or amino acid sequence(s) (Continuation of item 1.c of the first sheet) |
| --- |

1. With regard to any nucleotide and/or amino acid sequence disclosed in the international application, the international search was carried out on the basis of a sequence listing:

    a.    ☑   forming part of the international application as filed:

           ☑   in the form of an Annex C/ST.25 text file.

           ☐   on paper or in the form of an image file.

    b.    ☐   furnished together with the international application under PCT Rule 13*ter*.1(a) for the purposes of international search only in the form of an Annex C/ST.25 text file.

    c.    ☐   furnished subsequent to the international filing date for the purposes of international search only:

           ☐   in the form of an Annex C/ST.25 text file (Rule 13*ter*.1(a)).

           ☐   on paper or in the form of an image file (Rule 13*ter*.1(b) and Administrative Instructions, Section 713).

2.   ☐   In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

    [1]   The actually submitted sequence table is an XML file of the ST.26 standard.

**INTERNATIONAL SEARCH REPORT**
**Information on patent family members**

International application No.

**PCT/CN2022/130701**

| Patent document cited in search report | | | Publication date (day/month/year) | Patent family member(s) | | | Publication date (day/month/year) |
|---|---|---|---|---|---|---|---|
| CN | 110177808 | A | 27 August 2019 | IL | 267902 | A | 26 September 2019 |
| | | | | TW | 201831505 | A | 01 September 2018 |
| | | | | MX | 2019008503 | A | 13 September 2019 |
| | | | | EP | 3571229 | A1 | 27 November 2019 |
| | | | | SG | 10202107808 S | A | 30 August 2021 |
| | | | | JP | 2020503885 | A | 06 February 2020 |
| | | | | BR | 112019014615 | A2 | 02 June 2020 |
| | | | | WO | 2018136570 | A1 | 26 July 2018 |
| | | | | SG | 11201906468 T | A | 27 August 2019 |
| | | | | KR | 20190130559 | A | 22 November 2019 |
| | | | | CA | 3049674 | A1 | 26 July 2018 |
| | | | | US | 2019367621 | A1 | 05 December 2019 |
| | | | | AU | 2018211081 | A1 | 18 July 2019 |
| CN | 111629734 | A | 04 September 2020 | BR | 112020005938 | A2 | 17 November 2020 |
| | | | | EP | 3687553 | A1 | 05 August 2020 |
| | | | | SG | 11202002321 Y | A | 29 April 2020 |
| | | | | CA | 3075806 | A1 | 04 April 2019 |
| | | | | ZA | 202002046 | B | 26 October 2022 |
| | | | | AU | 2018338647 | A1 | 07 May 2020 |
| | | | | WO | 2019067805 | A1 | 04 April 2019 |
| | | | | JP | 2021500861 | A | 14 January 2021 |
| | | | | EA | 202090839 | A1 | 04 February 2021 |
| | | | | KR | 20200071079 | A | 18 June 2020 |
| | | | | IL | 273201 | A | 30 April 2020 |
| CN | 110483639 | A | 22 November 2019 | US | 2021214447 | A1 | 15 July 2021 |
| | | | | CA | 3100260 | A1 | 21 November 2019 |
| | | | | CN | 110770256 | A | 07 February 2020 |
| | | | | WO | 2019218944 | A2 | 21 November 2019 |
| | | | | EP | 3808773 | A2 | 21 April 2021 |
| | | | | JP | 2021530436 | A | 11 November 2021 |
| CN | 112426438 | A | 02 March 2021 | None | | | |

Form PCT/ISA/210 (patent family annex) (January 2015)

**EP 4 431 523 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5399346 A
- US 5580859 A
- US 5589466 A
- WO 0196584 A
- WO 0129058 A
- US 6326193 B
- US 5350674 A
- US 5585362 A

**Non-patent literature cited in the description**

- **UI-TEI et al.** *FEBS Letters,* 2000, vol. 479, 79-82
- **SAMBROOK et al.** Molecular Cloning: A Laboratory Manual. Cold Spring Harbor Laboratory, 2001